# EUROPÄISCHE PATENTANMELDUNG

(11) **EP 4 760 327 A1**
(43) Veröffentlichungstag der Anmeldung: **17.06.2026**
(21) Anmeldenummer: 24219665.7
(22) Anmeldetag: 13.12.2024
(51) Int. Cl.: G01R 33/48, A61B 5/00, A61B 5/055, A61B 6/04, A61B 6/00, A61B 6/03

(54) **KOMBINIERTE BILDGEBUNGSVORRICHTUNG MIT EINER POSITIONSBESTIMMUNGSEINHEIT ZU EINER BESTIMMUNG EINER POSITION EINES PATIENTENTISCHS**

(71) Anmelder: Siemens Healthineers AG, 91301 Forchheim (DE)
(72) Erfinder: Schröter, Steffen, 90763 Fürth (DE); Sukkau, Johann, 91074 Herzogenaurach (DE)
(74) Vertreter: Siemens Healthineers Patent Attorneys

(57) **Zusammenfassung**

Die Erfindung geht aus von einer kombinierten Bildgebungsvorrichtung, die eine erste medizinische Bildgebungsvorrichtung, die als Magnetresonanzvorrichtung ausgebildet ist, und eine weitere medizinische Bildgebungsvorrichtung umfasst, mit einen Patientenaufnahmebereich, einer Patientenlagerungsvorrichtung, die einen in zumindest eine Richtung bewegbaren Patiententisch aufweist, und einer Positionsbestimmungseinheit zu einer Bestimmung einer Position des Patiententischs in zumindest eine Richtung, wobei die Positionsbestimmungseinheit eine Erfassungseinheit, die an einer den Patientenaufnahmebereich umgebenden Umhausung angeordnet ist, und eine Positionskodiereinheit, die an dem Patiententisch angeordnet ist, aufweist, wobei die Erfassungseinheit zur Bestimmung der Position des Patiententischs eine Information der Positionskodiereinheit erfasst.

## Beschreibung

Die vorliegende Erfindung betrifft eine kombinierte Bildgebungsvorrichtung, die eine erste medizinische Bildgebungsvorrichtung, die als Magnetresonanzvorrichtung ausgebildet ist, und eine weitere medizinische Bildgebungsvorrichtung umfasst, mit einem Patientenaufnahmebereich, einer Patientenlagerungsvorrichtung, die einen in zumindest eine Richtung bewegbaren Patiententisch aufweist, und einer Positionsbestimmungseinheit zu einer Bestimmung einer Position des Patiententischs in zumindest eine Richtung.

Unabhängig vom grammatikalischen Geschlecht eines bestimmten Begriffes sind Personen mit männlicher, weiblicher oder anderer Geschlechteridentität mit umfasst.

Bei kombinierten Bildgebungsvorrichtungen, beispielsweise einer Bildgebungsvorrichtung mit einer Magnetresonanzvorrichtung und einer PET-Vorrichtung (Positron-Emissions-Tomographie-Vorrichtung) oder einer Röntgen-Vorrichtung, ist es wichtig, dass eine genaue Position des Patiententischs und damit des zu untersuchenden Bereichs des Patienten für die unterschiedlichen bildgebenden Untersuchungen vorliegt. Wünschenswert ist dabei, die Position des Patiententischs, insbesondere des zu untersuchenden Bereichs des Patienten, in mehr als nur einer Raumrichtung zu erhalten.

Zur Bestimmung der Position des Patiententischs bei Bildgebungsvorrichtungen mit einer Magnetresonanzvorrichtung ist es bisher bekannt, dass Seilzugsysteme mit einem entsprechenden Encoder verwendet werden. Dabei kommt ein Seilzugsystem zum Einsatz, der entweder nur einer Genauigkeit von 0,5 mm bis 1,0 mm bei der Positionsbestimmung liefern oder die Position nur innerhalb der Patientenlagerungsvorrichtung und nicht bezüglich eines Patientenaufnahmebereichs und/oder einer Scannereinheit der Bildgebungsvorrichtung bestimmen kann. Zudem weist ein derartiges Seilzugsystem einen weiteren Nachteil auf, dass nämlich nur in einer Raumrichtung, insbesondere in die Einfahrrichtung des Patiententischs in den Patientenaufnahmebereich, eine Positionsbestimmung erfolgt, und für die weiteren Raumrichtungen stets von einer Idealposition ausgegangen wird. Jedoch weist bei einem Einfahren des Patiententischs in den Patientenaufnahmebereich der Patiententisch in x-Richtung eine Toleranz von ca. 1 mm auf, so dass auch hier eine gewisse Ungenauigkeit vorliegen kann. Des Weiteren kann sich auch der Patiententisch bei schweren Patienten durchbiegen und damit auch in y-Richtung eine andere Position einnehmen als bei leichten Patienten oder bei einem leeren Patiententisch.

Der vorliegenden Erfindung liegt insbesondere die Aufgabe zugrunde, eine möglichst genaue Bestimmung einer Position des Patiententischs bezüglich einer Scannereinheit einer kombinierten Bildgebungsvorrichtung bereitzustellen. Die Aufgabe wird durch die Merkmale des unabhängigen Anspruchs gelöst. Vorteilhafte Ausgestaltungen sind in den Unteransprüchen beschrieben.

Die Erfindung geht aus von einer kombinierten Bildgebungsvorrichtung, die eine erste medizinische Bildgebungsvorrichtung, die als Magnetresonanzvorrichtung ausgebildet ist, und eine weitere medizinische Bildgebungsvorrichtung umfasst, mit einen Patientenaufnahmebereich, einer Patientenlagerungsvorrichtung, die einen in zumindest eine Richtung bewegbaren Patiententisch aufweist, und einer Positionsbestimmungseinheit zu einer Bestimmung einer Position des Patiententischs in zumindest eine Richtung. Erfindungsgemäß wird vorgeschlagen, dass die Positionsbestimmungseinheit eine Erfassungseinheit, die an einer den Patientenaufnahmebereich umgebenden Umhausung angeordnet ist, und eine Positionskodiereinheit, die an dem Patiententisch angeordnet ist, aufweist, wobei die Erfassungseinheit zur Erfassung der Position des Patiententischs eine Positionsinformation der Positionskodiereinheit erfasst.

Die kombinierte Bildgebungsvorrichtung umfasst bevorzugt zwei unterschiedliche bildgebende medizinische Modalitäten und/oder zwei unterschiedliche medizinische Bildgebungsvorrichtungen, die zu unterschiedlichen Untersuchungen eines zu untersuchenden Bereichs eines Patienten ausgebildet sind. Eine erste medizinische Modalität und/oder eine erste medizinische Bildgebungsvorrichtung umfasst dabei eine Magnetresonanzvorrichtung. Eine zweite medizinische Modalität und/oder eine zweite medizinische Bildgebungsvorrichtung umfasst eine von einer Magnetresonanzvorrichtung unterschiedliche medizinische Modalität und/oder unterschiedliche medizinische Bildgebungsvorrichtung. Die zweite medizinische Modalität und/oder die zweite medizinische Bildgebungsvorrichtung kann dabei eine PET-Vorrichtung oder eine Röntgenvorrichtung und/oder weitere, dem Fachmann als sinnvoll erscheinende medizinische Bildgebungsvorrichtung umfassen.

Die Magnetresonanzvorrichtung umfasst bevorzugt eine medizinische und/oder diagnostische Magnetresonanzvorrichtung, die zu einem Erfassen von medizinischen und/oder diagnostischen Bilddaten, insbesondere medizinischen und/oder diagnostischen Magnetresonanzbilddaten, eines Patienten ausgelegt und/oder ausgebildet ist. Die Magnetresonanzvorrichtung umfasst hierzu eine als Magneteinheit ausgebildete Scannereinheit zur Erfassung der medizinischen und/oder diagnostischen Bilddaten. Bevorzugt umfasst hierbei die Magneteinheit einen Grundmagneten, eine Gradienteneinheit und eine Hochfrequenzantenneneinheit. Die Hochfrequenzantenneneinheit ist fest innerhalb der Magneteinheit angeordnet und zur Aussendung von Anregungspulsen, insbesondere von Hochfrequenzpulsen, ausgelegt und/oder ausgebildet.

Der Grundmagnet ist zur Erzeugung eines homogenen Grundmagnetfelds mit einer definierten Magnetfeldstärke, wie beispielsweise mit einer Magnetfeldstärke von 3 T oder 1,5 T usw., ausgebildet. Insbesondere ist der Grundmagnet zur Erzeugung eines starken und konstanten Grundmagnetfelds ausgebildet. Das homogene Grundmagnetfeld ist bevorzugt innerhalb des Patientenaufnahmebereichs der Magnetresonanzvorrichtung angeordnet und/oder vorzufinden. Die Gradienteneinheit ist zu einer Erzeugung von Magnetfeldgradienten, die für eine Ortskodierung während einer Bildgebung verwendet werden, ausgebildet.

Die PET-Vorrichtung umfasst mehrere Positronen-Emissions-Tomographie-Detektormodule (PET-Detektormodule), die bevorzugt zu einer Ringform angeordnet sind und den Patientenaufnahmebereich in der Umfangsrichtung umgeben. Die PET-Detektormodule können jeweils mehrere Positronen-Emissions-Tomographie-Detektorelemente (PET-Detektorelemente) aufweisen, die zu einem PET-Detektorarray angeordnet sind, das ein Szintillationsdetektorarray mit Szintillationskristallen, beispielsweise LSO-Kristalle, umfasst. Des Weiteren umfassen die PET-Detektormodule bevorzugt jeweils ein Photodiodenarray, beispielsweise Avalanche-Photodiodenarray oder APD-Photodiodenarray, die dem Szintillationsdetektorarray nachgeschaltet innerhalb der PET-Detektormodule angeordnet sind.

Mittels der PET-Detektormodule werden Photonenpaare, die aus der Annihilation eines Positrons mit einem Elektron resultieren, erfasst. Das Positron wird hierbei von einem Radiopharmakon emittiert, wobei das Radiopharmakon über eine Injektion dem Patienten verabreicht wird. Trajektorien der beiden Photonen schließen dabei einen Winkel von 180° ein. Zudem weisen die beiden Photonen jeweils eine Energie von 511 keV auf. Beim Durchlaufen von Materie im Strahlengang können die bei der Annihilation entstandenen PET-Photonen abgeschwächt werden, wobei die Abschwächungswahrscheinlichkeit von der Pfadlänge durch die Materie und dem entsprechenden Abschwächungskoeffizienten der Materie abhängt.

Die Röntgenvorrichtung weist bevorzugt eine Röntgenquelle und einem Röntgendetektor auf und ist zur Aufnahme von Röntgenbilddaten eines Organs oder Körperteils des Patienten ausgebildet.

Die beiden medinischen Bildgebungsvorrichtungen können dabei derart ausgebildet sein, dass eine Scannereinheit der zweiten medizinischen Bildgebungsvorrichtung in eine Scannereinheit der Magnetresonanzvorrichtung integriert ist und nur ein einziges Gerät mit einem einzigen Patientenaufnahmebereich für eine Untersuchung des Patienten, insbesondere der zu untersuchenden Bereichs des Patienten, zur Verfügung steht. Zudem können die beiden medizinischen Bildgebungsvorrichtung zwei getrennte Geräte umfassen, wobei der Patient für eine Untersuchung zwischen den beiden medizinischen Geräten umpositioniert werden muss.

Der Patientenaufnahmebereich ist zu einer Aufnahme des Patienten, insbesondere des zu untersuchenden Bereichs des Patienten, für eine medizinische Magnetresonanzuntersuchung und einer weiteren medizinischen Bildgebungsuntersuchung ausgelegt und/oder ausgebildet. Der Patientenaufnahmebereich umfasst bevorzugt den Bereich, der dem Patienten während den medizinischen Bildgebungsuntersuchungen zur Verfügung steht. Beispielsweise ist hierzu der Patientenaufnahmebereich zylinderförmig ausgebildet und/oder zylinderförmig von der Magneteinheit der Magnetresonanzvorrichtung und/oder zylinderförmig von der Scannereinheit der weiteren Bildgebungsvorrichtung umgeben. Bevorzugt umfasst hierbei die Magneteinheit und/oder der Scannereinheit der weiteren Bildgebungsvorrichtung eine den Patientenaufnahmebereich zumindest teilweise umgebende Umhausung. Dabei umgibt die Umhausung den Patientenaufnahmebereich zylinderförmig.

Innerhalb des Patientenaufnahmebereichs ist bevorzugt ein Field of View (FOV) und/oder ein Isozentrum der kombinierten Bildgebungsvorrichtung angeordnet. Das FOV umfasst bevorzugt einen Erfassungsbereich der Magnetresonanzvorrichtung und/oder der weiteren Bildgebungsvorrichtung, innerhalb dessen die Bedingungen für eine Erfassung von medizinischen Bilddaten, beispielsweise von Magnetresonanzbilddaten und/oder PET-Bilddaten und/oder Röntgenbilddaten, innerhalb des Patientenaufnahmebereichs vorliegen. Beispielsweise umfasst das FOV ein homogenes Grundmagnetfeld einer Magnetresonanzvorrichtung und/oder einen Sichtbereich der PET-Detektormodule und/oder einen Sichtbereich eines Röntgendetektors. Das Isozentrum der kombinierten Bildgebungsvorrichtung umfasst bevorzugt den Bereich und/oder Punkt innerhalb der kombinierten Bildgebungsvorrichtung, der die optimalen und/oder idealen Bedingungen für die Erfassung von medizinischen Bilddaten aufweist. Beispielsweise umfasst das Isozentrum den homogensten Magnetfeldbereich innerhalb der Magnetresonanzvorrichtung.

Für eine Positionierung des Patienten, insbesondere des zu untersuchenden Bereichs des Patienten, innerhalb des Patientenaufnahmebereich weist die kombinierte Bildgebungsvorrichtung die Patientenlagerungsvorrichtung auf. Die Patientenlagerungsvorrichtung ist zu einer Lagerung des Patienten während der kombinierten medizinischen Bildgebungsuntersuchung ausgebildet. Die Patientenlagerungsvorrichtung weist bevorzugt einen bewegbaren Patiententisch auf, der insbesondere innerhalb des Patientenaufnahmebereichs der kombinierten Bildgebungsvorrichtung bewegbar ausgebildet ist. Bevorzugt ist hierbei der Patiententisch in Längsrichtung des Patientenaufnahmebereichs innerhalb des Patientenaufnahmebereichs bewegbar ausgebildet. Die Längsrichtung des Patientenaufnahmebereichs ist bevorzugt parallel zu einer z-Richtung der kombinierten Bildgebungsvorrichtung ausgerichtet. Bei einem Einfahren des Patiententischs in den Patientenaufnahmebereich wird der Patiententisch in z-Richtung auf einer Führungsschiene geführt. Hierbei weist die Bewegung eine Toleranz in x-Richtung der kombinierten Bildgebungsvorrichtung von ca. 1 mm auf. Die x-Richtung der kombinierten Bildgebungsvorrichtung ist dabei senkrecht zur z-Richtung und senkrecht zu einer auf den Patiententisch wirkenden Gewichtskraft ausgerichtet. Des Weiteren kann sich auch der Patiententisch bei einer Positionierung eines schweren Patienten auf dem Patiententisch stärker durchbiegen als bei einer Positionierung eines leichten Patienten auf dem Patiententisch oder einem leeren Patiententisch. Somit kann auch in y-Richtung der kombinierten Bildgebungsvorrichtung eine Position des Patiententischs variieren. Die y-Richtung der kombinierten Bildgebungsvorrichtung ist dabei senkrecht zur z-Richtung und senkrecht zur x-Richtung der kombinierten Bildgebungsvorrichtung ausgerichtet. Bevorzugt ist die y-Richtung der kombinierten Bildgebungsvorrichtung parallel zu der auf den Patiententisch wirkenden Gewichtskraft ausgerichtet.

Zu einer Bestimmung einer Position des Patiententischs in zumindest eine Richtung weist die kombinierte Bildgebungsvorrichtung die Positionsbestimmungseinheit auf. Die Bestimmung der Position des Patiententischs erfolgt bevorzugt bezüglich eines Referenzpunkts, der von dem Patientenaufnahmebereich und/oder zumindest einer der Scannereinheiten umfasst ist. Beispielsweise kann der Referenzpunkt eine Öffnung des Patientenaufnahmebereichs umfassen. Alternativ oder zusätzlich kann der Referenzpunkt auch das Isozentrum der Magnetresonanzvorrichtung und/oder das Isozentrum der weiteren Bildgebungsvorrichtung umfassen.

Die Positionsbestimmungseinheit weist die Erfassungseinheit und die Positionskodiereinheit auf. Die Positionskodiereinheit ist bevorzugt dazu ausgebildet, eine eindeutige Kodierung für unterschiedliche Positionen des Patiententischs bereitzustellen, so dass die unterschiedlichen Positionen des Patiententischs anhand ihrer Kodierung unterschieden werden können. Die Positionskodiereinheit ist dabei am Patiententisch angeordnet. Bevorzugt weist die Positionskodiereinheit eine Länge auf, die einer Länge des Patiententischs entspricht. Insbesondere bewegt sich die Positionskodiereinheit mit dem Patiententisch mit, so dass jeder Position entlang der Länge des Patiententischs eine eindeutige Kodierung mittels der Positionskodiereinheit zugeordnet werden kann. Die Positionsinformation der Positionskodiereinheit umfasst bevorzugt eine Positionskodierung.

Die Positionskodiereinheit kann dabei eine Kodierung aufweisen, die neben einer Positionskodierung in eine erste Richtung auch eine Positionskodierung in eine zweite Richtung, wobei die zweite Richtung bevorzugt senkrecht zur ersten Richtung ausgerichtet ist. Besonders vorteilhaft ist hierbei die erste Richtung in z-Richtung ausgerichtet und die zweite Richtung in x-Richtung und/oder in y-Richtung ausgerichtet.

Die Erfassungseinheit ist zur Erfassung der Positionsinformation der Positionskodiereinheit ausgebildet. Bevorzugt weist hierzu die Erfassungseinheit eine Sensoreinheit, beispielsweise eine Kamera und/oder eine optische Sensoreinheit auf. Die Erfassungseinheit ist an der den Patientenaufnahmebereich umgebenden Umhausung angeordnet, so dass bei einer Bewegung des Patiententischs eine Relativbewegung zwischen der Positionskodiereinheit und der Erfassungseinheit erfolgt. Bevorzugt ist ein Erfassungsbereich der Erfassungseinheit auf den Patiententisch, insbesondere auf den Bereich des Patiententischs, an dem die Positionskodiereinheit angeordnet ist, gerichtet.

Bevorzugt werden zu einer Auswertung der mittels der Erfassungseinheit erfassten Positionsinformationen der Positionskodiereinheit an eine Auswerteeinheit weitergeleitet. Die Auswerteeinheit kann dabei von einer Steuereinheit und/oder Recheneinheit umfasst sein. Dabei kann die Steuereinheit und/oder Recheneinheit von der Positionsbestimmungseinheit umfasst sein oder auch in eine Systemsteuereinheit der kombinierten Bildgebungsvorrichtung integriert sein.

Durch die Erfindung kann vorteilhaft erreicht werden, dass jede Positionsänderung des Patiententischs bezüglich des Patientenaufnahmebereichs erfasst werden kann. Insbesondere kann hierbei die exakte Position des Patiententischs in zumindest eine Richtung erfasst werden. Die exakte Position des Patiententischs ist bei kombinierten Bildgebungssystemen besonders wichtig für eine Übereinstimmung des zu untersuchenden Bereichs für die unterschiedlichen medizinischen Bildgebungsuntersuchungen. Zudem ist auch bei kombinierten Bildgebungsvorrichtungen mit einer PET-Vorrichtung eine exakte Position des Patiententischs für eine Berechnung und Bereitstellung einer Schwächungskorrekturkarte (µ-Map) bei der Erfassung und Bestimmung einer Energie der erfassten Photonen von großer Bedeutung.

In einer vorteilhaften Weiterbildung der erfindungsgemäßen kombinierten Bildgebungsvorrichtung kann es vorgesehen sein, dass die Positionskodiereinheit ein Kodierband umfasst, das an einer Seite des Patiententischs angeordnet ist, wobei die Seite keine zur Lagerung eines Patienten ausgebildete Fläche umfasst. Dabei weist das Kodierband bevorzugt eine Länge des Patiententischs auf, so dass für unterschiedliche Längenabschnitte des gesamtem Patiententischs eine Positionsinformation zur Erfassung bereitgestellt werden kann. Das Kodierband kann beispielsweise eine Länge von 2200 mm umfassen. Zudem kann das Kodierband eine Breite von beispielsweise 30 mm und eine Dicke von beispielsweise 3 mm umfassen. Das Kodierband kann dabei beispielsweise ein Kunststoffband mit einem PVC-Material und/oder weiteren, dem Fachmann als sinnvoll erscheinende Materialien umfassen. Bevorzugt umfasst das Kodierband für unterschiedliche Längenabschnitte des Patiententischs unterschiedliche Positionsinformationen.

Die Seite des Patiententischs, an dem das Kodierband angeordnet ist, umfasst dabei bevorzugt eine Unterseite des Patiententischs und/oder eine Seitenfläche des Patiententischs. Die Seite des Patiententischs, die eine Oberfläche zur Lagerung des Patienten aufweist, umfasst eine Oberseite des Patiententischs. Dabei kann das Kodierband auf die Unterseite des Patiententischs oder auf eine Seitenfläche des Patiententischs geklebt sein. Durch die Anordnung der Positionskodiereinheit an einer Seitenfläche des Patiententischs und/oder an der Unterseite des Patiententischs kann eine geschützte Anordnung der Positionskodiereinheit vorteilhaft erreicht werden.

In einer vorteilhaften Weiterbildung der erfindungsgemäßen kombinierten Bildgebungsvorrichtung kann es vorgesehen sein, dass die Positionskodiereinheit eine Kodierung mit mehreren Kodierstrukturen umfasst, wobei die einzelnen Kodierstrukturen jeweils eine Positionsinformation in z-Richtung des Patiententischs und/oder eine Positionsinformation in eine weitere Richtung des Patiententischs umfassen. Bevorzugt sind die mehreren Kodierstrukturen derart ausgebildet, dass jede der Kodierstrukturen sich von den restlichen Kodierstrukturen in zumindest einem Strukturmerkmal unterscheidet, so dass jede Position in die zumindest eine Richtung des Patiententischs durch eine der mehreren Kodierstrukturen eindeutig definiert ist. Zudem können die unterschiedlichen Kodierstrukturen auch derart ausgebildet sein, dass in zumindest zwei Richtungen, insbesondere in die z-Richtung und eine weitere Richtung, des Patiententischs eine eindeutige Positionszuordnung erfolgen kann. Die Positionsinformation in eine weitere Richtung des Patiententischs umfasst insbesondere die x-Richtung oder die y-Richtung des Patiententischs. Derart kann durch Erfassen einer Kodierstruktur eine eindeutige Bestimmung einer Position des Patiententischs in die zumindest eine Richtung bereitgestellt werden.

In einer vorteilhaften Weiterbildung der erfindungsgemäßen kombinierten Bildgebungsvorrichtung kann es vorgesehen sein, dass die einzelnen Kodierstrukturen jeweils eine Positionskodierung, die einen definierten Längenabschnitt des Patiententischs zugeordnet ist, und eine Informationskodierung, umfassen. Die Positionskodierungen umfassen bevorzugt eine zweidimensionale Struktur. Insbesondere können die einzelnen Positionskodierungen eine Positionsinformation in z-Richtung und eine weitere Richtung, insbesondere in x-Richtung und/oder in y-Richtung, des Patiententischs umfassen. Die Informationskodierung stellt dabei eine zusätzliche Information zur Positionskodierung bereit. Diese zusätzliche Information kann beispielsweise eine Eindeutigkeit der Positionskodierung für einen Längenabschnitt des Patiententischs umfassen.

Eine Anzahl der Längenabschnitte und/oder eine Größe der Längenabschnitte des Patiententischs, insbesondere einer Länge des Patiententischs in z-Richtung, kann dabei abhängig sein von einer Struktur der Kodierung, insbesondere eine Größe einer Struktur der Kodierung, insbesondere in z-Richtung des Patiententischs. Alternativ oder zusätzlich kann die Größe der Längenabschnitte des Patiententischs, insbesondere einer Länge des Patiententischs in z-Richtung, auch abhängig sein von einer Auflösung der Erfassungseinheit zur Erfassung der Kodierstruktur. Bevorzugt weist ein Längenabschnitt des Patiententischs, insbesondere der Länge des Patiententischs in z-Richtung, maximal 20 mm auf. Bevorzugt weist ein Längenabschnitt des Patiententischs, insbesondere der Länge des Patiententischs in z-Richtung, maximal 19 mm auf. Bevorzugt weist ein Längenabschnitt des Patiententischs, insbesondere der Länge des Patiententischs in z-Richtung, maximal 18 mm auf. Bevorzugt weist ein Längenabschnitt des Patiententischs, insbesondere der Länge des Patiententischs in z-Richtung, maximal 17 mm auf. Bevorzugt weist ein Längenabschnitt des Patiententischs, insbesondere der Länge des Patiententischs in z-Richtung, maximal 16 mm auf. Bevorzugt weist ein Längenabschnitt des Patiententischs, insbesondere der Länge des Patiententischs in z-Richtung, maximal 15 mm auf. Bevorzugt weist ein Längenabschnitt des Patiententischs, insbesondere der Länge des Patiententischs in z-Richtung, maximal 14 mm auf. Bevorzugt weist ein Längenabschnitt des Patiententischs, insbesondere der Länge des Patiententischs in z-Richtung, maximal 13 mm auf. Bevorzugt weist ein Längenabschnitt des Patiententischs, insbesondere der Länge des Patiententischs in z-Richtung, maximal 12 mm auf. Bevorzugt weist ein Längenabschnitt des Patiententischs, insbesondere der Länge des Patiententischs in z-Richtung, maximal 11 mm auf. Bevorzugt weist ein Längenabschnitt des Patiententischs, insbesondere der Länge des Patiententischs in z-Richtung, maximal 10 mm auf. Bevorzugt weist ein Längenabschnitt des Patiententischs, insbesondere der Länge des Patiententischs in z-Richtung, maximal 9 mm auf. Bevorzugt weist ein Längenabschnitt des Patiententischs, insbesondere der Länge des Patiententischs in z-Richtung, maximal 8 mm auf. Bevorzugt weist ein Längenabschnitt des Patiententischs, insbesondere der Länge des Patiententischs in z-Richtung, maximal 7 mm auf. Bevorzugt weist ein Längenabschnitt des Patiententischs, insbesondere der Länge des Patiententischs in z-Richtung, maximal 6 mm auf. Bevorzugt weist ein Längenabschnitt des Patiententischs, insbesondere der Länge des Patiententischs in z-Richtung, maximal 5 mm auf. Jede dieser Längeneinheiten ist durch eine Kodierstruktur definiert und/oder festgelegt, so dass bei einer Erfassung der entsprechenden Kodierstruktur auch die entsprechende Position des Patiententischs am Erfassungsort ermittelt werden kann.

In einer vorteilhaften Weiterbildung der erfindungsgemäßen kombinierten Bildgebungsvorrichtung kann es vorgesehen sein, dass die Kodierung einen Binärcode umfasst, wobei der Binärcode eine 8-Bit-Kodierung mit einer 2x4-Codematrix oder eine 9-Bit-Kodierung mit einer 3x3-Codematrix oder eine 9-Bit-Kodierung mit einer 3x4-Codematrix umfasst. Bevorzugt weist die Positionskodierung den Binärcode mit der 8-Bit-Kodierung in einer 2x4-Codematrix oder eine 9-Bit-Kodierung in einer 3x3-Codematrix oder eine 9-Bit-Kodierung in einer 3x4-Codematrix auf.

Dabei umfasst die 2x4-Codematrix eine Matrix mit zwei Reihen und vier Spalten, in der der Binärcode auf die zwei Reihen aufgeteilt dargestellt ist. Der 8-Bit-Kodierung stehen dabei 255 unterschiedliche Längenkodierungen zur Einteilung der Länge des Patiententischs in Längenabschnitte zur Verfügung.

Der 9-Bit-Kodierung stehen dabei 511 unterschiedliche Längenkodierungen zur Einteilung der Länge des Patiententischs in Längenabschnitte zur Verfügung. Die 3x3-Codematrix umfasst eine Matrix mit drei Reihen und drei Spalten, in der der Binärcode auf die drei Reihen aufgeteilt ist. Die 3x4-Codematrix umfasst eine Matrix mit drei Reihen und vier Spalten, in der der Binärcode auf die drei Reihen aufgeteilt ist, wobei die letzte Reihe mit nur einem Bit für die 9-Bit-Kodierung versehen ist und die restlichen drei Bits zusätzliche Informationen bezüglich Eindeutigkeit der Kodierung für eine zuverlässige Erfassung der Kodierung aufweisen können.

Der Binärcode ist dabei von dem Kodierband umfasst, das am Patiententisch angeordnet ist. Die Kodierung, insbesondere die Binärkodierung kann dabei in das Kodierband eingefräst und/oder eingebohrt angeordnet sein. Beispielsweise können für jeden Bit der Kodierung, sofern dieser Bit den Wert 1 umfasst, eine Ausnehmung in das Kodierband eingefräst oder eingebohrt sein. Eine derartige Ausnehmung kann in der einfachsten Ausgestaltung eine Form einer Kreisfläche umfassen. Das Fräsen oder Bohren der einzelnen Kodierungen kann dabei mittels einer CNC-Fräse oder weiteren, dem Fachmann als sinnvoll erscheinenden Geräten erfolgen. Eine derartige Ausnehmung in Form einer Kreisfläche kann beispielsweise einen Durchmesser 1 mm oder 2 mm aufweisen. Für eine bessere Sichtbarkeit der Ausnehmungen und/oder zur Verstärkung eines Kontrasts zwischen den Ausnehmungen und dem Kodierband, sind die Ausnehmungen zusätzlich mit einer Farbe gefüllt. Bevorzugt weist die Farbe zur Füllung der Ausnehmungen einen hohen farblichen Kontrast zu einer Farbe des Kodierbands auf. Weist das Kodierband eine beispielsweise weiße Farbe auf, werden bevorzugt die Ausnehmungen mit einer schwarzen Farbe gefüllt. Zudem ist es auch denkbar, für unterschiedliche Ausnehmungen Füllungen mit unterschiedlichen Farben zu verwenden.

Die Farbe zum Befüllen der Ausnehmungen kann beispielsweise ein 2-Komponenten Epoxidharz mit schwarzen Pigmenten umfassen. Nach einem Befüllen der Ausnehmungen mit Farbe und eine anschließendem Trocknen und Polieren erhält man einen großen Kontrast zu dem Kodierband. Im Gegensatz zu einem digitalen Druckverfahren weisen die mit Farbe befüllten Ausnehmungen keine Pixelstruktur auf, so dass eine Genauigkeit bei der Erfassung der Kodierung nur von einer Auflösung der Erfassungseinheit abhängt.

Beispielsweise sind bei einer 8-Bit-Kodierung in einer 2x4-Codematrix nur die Ausnehmungen vorhanden und mit Farbe befüllt, die den Wert "1" aufweisen und die Bitplätze, die den Wert "0" aufweisen, bleiben leer. Entsprechend verhält es sich auch bei einer 9-Bit-Kodierung mit einer 3x3-Codematrix oder eine 9-Bit-Kodierung mit einer 3x4-Codematrix.

Diese Ausgestaltung der Erfindung ermöglicht eine einfache und effiziente Kodierung von Längenabschnitten einer Länge des Patiententischs. Zudem kann derart auf einfache Art und Weise eine aktuelle Position des Patiententischs bezüglich des Patientenaufnahmebereichs und/oder des Isozentrums und/oder der Scannereinheit erfasst und bestimmt werden.

In einer vorteilhaften Weiterbildung der erfindungsgemäßen kombinierten Bildgebungsvorrichtung kann es vorgesehen sein, dass die Kodierung zusätzlich einen Richtungscode und/oder einen Markierungscode und/oder einen Paritätscode umfasst. Bevorzugt weist die Informationskodierung den Richtungscode und/oder den Markierungscode und/oder den Paritätscode auf.

Der Richtungscode zeigt an, von welcher Seite und/oder Richtung die Kodierung gelesen werden muss, um die Positionsinformation der Kodierung zu erhalten. Beispielsweise kann sich der Richtungscode links von der Kodierung befinden und angeben, dass die Kodierung von links nach rechts gelesen werden muss. Der Richtungscode kann dabei ebenfalls zumindest eine Ausnehmung in dem Kodierband umfassen, wobei die Ausnehmung ebenfalls mit zu der Farbe des Kodierbands kontrastreichen Farbe gefüllt ist. Bevorzugterweise unterscheiden sich die Ausnehmungen des Richtungscodes in einer Größe von den Ausnehmungen der Positionskodierung des Patiententischs. Beispielsweise können die Ausnehmungen des Richtungscodes einen doppelt so großen Durchmesser aufweisen als eine Durchmesser der Ausnehmungen der Positionskodierung. Auch kann der Richtungscode mehrere mit Farbe gefüllte Ausnehmungen umfassen, die ein definiertes Muster umfassen und damit die Leserichtung für die Längenkodierung des Patiententischs festlegen. Zudem kann hierbei auch die Leserichtung für die Längenkodierung des Patiententischs festgelegt werden, egal mit welchem Endbereich der Patiententisch zuerst in den Patientenaufnahmebereich eingeführt wird.

Der Markierungscode gibt den Bereich an, der für eine Positionskodierung der einzelnen Längenabschnitte zur Verfügung steht. Damit erhöht der Markierungscode auch eine Sicherheit bei einer Erfassung der Positionskodierung. Ein derartiger Markierungscode kann dabei links und rechts neben der Positionskodierung angebracht sein. Insbesondere signalisiert der Markierungscode einen Anfang und ein Ende einer jeweiligen Positionskodierung. Der Markierungscode kann dabei ebenfalls zumindest eine Ausnehmung in dem Kodierband umfassen, wobei die Ausnehmung ebenfalls mit zu der Farbe des Kodierbands kontrastreichen Farbe gefüllt ist. Dabei können sich die Ausnehmungen des Markierungscodes ebenfalls in einer Größe von den Ausnehmungen der Positionskodierung des Patiententischs unterscheiden. Beispielsweise können die Ausnehmungen des Markierungscodes einen doppelt so großen Durchmesser aufweisen als eine Durchmesser der Ausnehmungen der Positionskodierung. Auch kann der Markierungscode mehrere mit Farbe gefüllte Ausnehmungen umfassen. Auch kann der Markierungscode einstückig und/oder einteilig mit dem Richtungscode ausgebildet sein.

Der Paritätscode zeigt an, ob die Positionskodierung eine gerade Zahl oder eine ungerade Zahl darstellen soll. Somit gibt der Paritätscode eine zusätzliche Sicherheit, die Positionskodierung korrekt erfasst zu haben. Der Paritätscode kann dabei eine 1-Bit-Kodierung umfassen, die bei gerader Zahl der Positionskodierung eine mit Farbe befüllte Ausnehmung umfasst und bei ungerader Zahl der Positionskodierung ein leeres Feld umfasst. Werden beispielswiese einzelnen Kodierfelder der Positionskodierung nicht erfasst, beispielsweise aufgrund von Verschmutzungen, dann kann diese zu einer Abweichung des Paritätscodes mit der Positionskodierung führen und die Erfassung als "Fehler" deklariert werden.

In einer vorteilhaften Weiterbildung der erfindungsgemäßen kombinierten Bildgebungsvorrichtung kann es vorgesehen sein, dass die Erfassungseinheit eine Kamera umfasst, wobei die Kamera eine Auflösung von mindestens 400 Pixeln in eine Erfassungsrichtung umfasst. Die Erfassungsrichtung umfasst bevorzugt diejenige Richtung, in der die Position des Patiententischs mittels der Kamera erfasst werden soll. Bevorzugt umfasst die Erfassungsrichtung die z-Richtung und eine weitere Richtung. Beispielsweise kann die Kamera eine VGA-Kamera umfassen mit einer Auflösung von 640x480 Pixeln aufweisen. Zudem können auch Kameras mit einer höheren Auflösung, beispielsweise von 1000 Pixeln in jede Erfassungsrichtung verwendet werden. Derart kann eine vorteilhafte hohe Genauigkeit bei der Erfassung einer Position des Patiententischs bereitgestellt werden. Weist beispielsweise die Erfassungseinheit, insbesondere eine Kamera der Erfassungseinheit, eine Auflösung von 500 Pixeln in z-Richtung und/oder in der Länge des Patiententischs auf, kann bei einer Längeneinheit von 15 mm eine Positionsgenauigkeit von 0,03 mm (=15mm/500px) bei der Erfassung der Position erreicht werden. Entspricht die Auflösung der Erfassungseinheit, insbesondere einer Kamera, 1000 Pixel in z-Richtung, ergibt sich bei einer gleichen Einteilung der Längeneinheiten des Patiententischs eine Positionsgenauigkeit von 0,015 mm.

In einer vorteilhaften Weiterbildung der erfindungsgemäßen kombinierten Bildgebungsvorrichtung kann es vorgesehen sein, dass die Erfassungseinheit weiterhin umfasst:
- eine Erfassungsoptik mit einem Prisma, wobei die Erfassungsoptik der Kamera vorgeschaltet angeordnet ist, und/oder
- zumindest eine LED und/oder
- zumindest einen Laser und/oder
- eine Steuereinheit.

Die Erfassungsoptik mit dem Prisma umfasst bevorzugt zusätzlich zu dem Prisma zumindest ein Objektiv, das die erfassten Informationen, insbesondere Positionsinformationen, auf die Kamera fokussiert. Bevorzugt ist hierbei das Objektiv entlang des Strahlengangs zwischen dem Prisma und der Kamera angeordnet. Mittels des Prismas, das dem Objektiv vorgeschaltet angeordnet ist, kann vorteilhaft Strahlengang der Erfassungseinheit gebrochen werden. Beispielsweise kann das Prisma ein 45°-Prisma umfassen, das derartig angeordnet ist, dass der Strahlengang der Erfassungseinheit um 90° mittels des Prismas gebrochen wird. Vorzugsweise umfasst dabei das Prisma eine Querschnittsfläche, die ein gleichschenkeliges rechtwinkliges Dreieck umfasst. Dies ermöglicht eine besonders platzsparende und geschützte Anordnung der Kamera, da diese, insbesondere eine Erfassungsrichtung der Kamera, parallel zu einer Längsrichtung des Patiententischs an der den Patientenaufnahmebereich umgebenden Umhausung angeordnet werden kann. Insbesondere benötigt hierdurch die Erfassungseinheit eine niedrige und platzsparende Bauhöhe zwischen der den Patientenaufnahmebereich umgebenden Umhausung und dem Patiententisch.

Die zumindest eine LED ist bevorzugt dazu ausgebildet, einen Kontrast bei der Erfassung der Positionskodierung zu verstärken. Bevorzugt weist die Erfassungseinheit mehr als eine LED auf, so dass Licht unterschiedlicher Wellenlängen zur Verstärkung eines Kontrasts, insbesondere eine Kontrast zwischen den einzelnen Kodierstrukturen und dem Kodierband, ausgesendet werden kann. Beispielsweise können die eine LED oder die mehreren LEDs weißes Licht oder Licht im Infrarot-Bereich (IR-Bereich) aussenden. Insbesondere wird mittels der zumindest einen LED eine homogene Ausleuchtung des von der Erfassungseinheit, insbesondere der Kamera, zu erfassenden Bereichs des Kodierbands ermöglicht. Dabei kann es vorgesehen sein, dass die zumindest eine LED von einer Steuereinheit derart angesteuert wird, dass nur dann ein Licht ausgesendet wird, wenn die Erfassungseinheit, insbesondere die Kamera, eine Positionserfassung mittels der Positionskodiereinheit durchführt.

Der zumindest eine Laser ist bevorzugt dazu ausgebildet, eine Positionsinformation für eine Erfassung durch die Erfassungseinheit in eine dritte Richtung des Patiententischs bereitzustellen. Die von dem zumindest einen Laser erzeugten Laserlinien und/oder Laserstrahlen sind dabei auf den Patiententisch gerichtet und werden dort reflektiert und von der Kamera erfasst. Bevorzugt werden dabei die von dem Laser erzeugten Laserlinien und/oder Laserstrahlen von der Erfassungsoptik auf die Kamera gelenkt. Der Laser ist dabei derart bezüglich des Patiententischs innerhalb der Erfassungseinheit positioniert, dass die Laserstrahlen und/oder die Laserlinien mit einem von 90° abweichenden Winkel auf den Patiententisch treffen. Bevorzugt treffen die Laserstrahlen und/oder die Laserlinien mit einen Winkel zwischen 30° und 60° auf den Patiententisch und werden dort reflektiert.

Eine Position des reflektierten Laserstrahls ist dabei abhängig von einem Abstand des Patiententischs in diese dritte Richtung, beispielsweise die y-Richtung oder die x-Richtung, zu dem zumindest einen Laser. Mittels einer Triangulationsberechnung kann aus den mittels der Kamera erfassten Laserstrahlen, insbesondere eine Reflexionsposition der Laserstrahlen, eine Position in die dritte Richtung des Patiententischs ermittelt werden. Ist beispielsweise die Erfassungseinheit unterhalb des Patiententischs an der den Patientenaufnahmebereich umgebenden Umhausung angeordnet, kann mittels des zumindest einen Lasers eine Positionsinformation in y-Richtung des Patiententischs bereitgestellt werden. Beispielsweise kann derart eine Durchbiegung des Patiententischs während einer Bildgebungsuntersuchung ermittelt werden. Dabei kann es vorgesehen sein, dass der zumindest eine Laser von einer Steuereinheit derart angesteuert wird, dass nur dann ein Laserstrahl ausgesendet wird, wenn die Erfassungseinheit, insbesondere die Kamera, eine Positionserfassung in die dritte Richtung durchführt.

Die Steuereinheit ist bevorzugt dazu ausgebildet, eine Erfassung einer Positionsinformation und ein Bestimmen einer Position des Patiententischs zu steuern. Insbesondere ist hierbei die Steuereinheit dazu ausgebildet, die zumindest eine LED und den zumindest einen Laser derart anzusteuern, dass diese nur in einem Betriebsmodus sind, wenn die jeweilige Erfassung der mit dem zumindest einen Laser oder der zumindest einen LED zusammenhängenden Positionsinformation von der Kamera erfasst wird. Zudem ist die Steuereinheit dazu ausgebildet, anhand der erfassten Positionsinformationen eine Position des Patiententischs zu Bestimmen. Die Steuereinheit kann hierzu auch eine Auswerteeinheit umfassen. Mittels der Steuereinheit kann eine schnelle Auswertung der erfassten Positionsinformationen ermöglicht werden. Insbesondere kann derart auch eine Auswertung der Positionsinformationen in Echtzeit bereitgestellt werden, so dass stets eine aktuelle Position des Patiententischs für eine Bestimmung einer Schwächungskorrektur und/oder eine Schwächungskorrekturkarte zur Verfügung steht.

Die Steuereinheit umfasst zumindest ein Rechenmodul und/oder einen Prozessor. So ist insbesondere die Steuereinheit dazu ausgebildet, computerlesbare Instruktionen auszuführen. Insbesondere umfasst die Steuereinheit eine Speichereinheit, wobei auf der Speichereinheit computerlesbare Informationen gespeichert sind, wobei die Steuereinheit dazu ausgebildet ist, die computerlesbaren Informationen von der Speichereinheit zu laden und die computerlesbaren Informationen auszuführen. Derart ist die Steuereinheit dazu ausgebildet, die eine Erfassung einer Position des Patiententischs in zumindest eine Richtung mittels der Erfassungseinheit, bevorzugt in drei Richtungen, und eine Bestimmung einer Position des Patiententischs in zumindest eine Richtung, bevorzugt in drei Richtungen, zu steuern.

Die Komponenten der Steuereinheit können zum überwiegenden Teil in Form von Softwarekomponenten ausgebildet sein. Grundsätzlich können diese Komponenten aber auch zum Teil, insbesondere wenn es um besonders schnelle Berechnungen geht, in Form von softwareunterstützten Hardwarekomponenten, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Selbstverständlich ist es auch denkbar, dass mehrere der genannten Komponenten in Form einer einzelnen Softwarekomponente bzw. softwareunterstützter Hardwarekomponente zusammengefasst realisiert sind.

Die Steuereinheit der Erfassungseinheit ist bevorzugt außerhalb des Patientenaufnahmebereichs und damit außerhalb des Schirmgehäuses angeordnet. Eine Datenübertragung innerhalb der Erfassungseinheit zwischen der Kamera und/oder einer Elektronikeinheit der Erfassungseinheit und der Steuereinheit und damit eine Datenübertragung zwischen einem Bereich innerhalb Schirmgehäuses, das innerhalb des Patientenaufnahmebereichs angeordnet ist, und einem Bereich außerhalb des Patientenaufnahmebereichs erfolgt bevorzugt über Kabel. Dabei können die Kabel beispielsweise Lichtwellenleiter und/oder weitere, dem Fachmann als sinnvoll erscheinende Kabel umfassen.

In einer vorteilhaften Weiterbildung der erfindungsgemäßen kombinierten Bildgebungsvorrichtung kann es vorgesehen sein, dass die Erfassungseinheit ein Schirmgehäuse mit einem Sichtfenster umfasst, wobei das Sichtfenster an einer dem Patientenaufnahmebereich und/oder dem Patiententisch zugewandten Seite des Schirmgehäuses angeordnet ist. Das Schirmgehäuse schirmt die Erfassungseinheit bezüglich einer Hochfrequenzstrahlung ab. Das Sichtfenster umfasst bevorzugt eine transparente Abdeckung, die eine Erfassung der Positionsinformationen ermöglicht. Bevorzugt weist das Sichtfenster, insbesondere die transparente Abdeckung ebenfalls eine bezüglich einer Hochfrequenzstrahlung abschirmende Eigenschaften auf. Beispielsweise kann die transparente Abdeckung aus Glas gebildet sein, bevorzugt einem bruchsicherem Glas. Für die abschirmende Eigenschaften weist das Glas bevorzugt eine transparente und elektrisch leitende Beschichtung auf. Beispielsweise kann eine derartige Beschichtung aus einem ITO-Material (Iridium-Zinn-Oxid-Material) gebildet sein. Alternativ oder zusätzlich ist es auch denkbar, dass die transparente Abdeckung eine Beschichtung, beispielsweise eine dünne leitende Schicht aus Silber, umfasst. Derart kann ein vorteilhafter Schutz der Erfassungseinheit bereitgestellt werden. Zudem kann eine unerwünschte Beeinträchtigung der Erfassungseinheit vorteilhaft verhindert werden.

In einer vorteilhaften Weiterbildung der erfindungsgemäßen kombinierten Bildgebungsvorrichtung kann es vorgesehen sein, dass die Erfassungseinheit zu einer Erfassung einer Positionsinformation in zumindest zwei Richtungen des Patiententischs ausgebildet ist. Umfasst die kombinierte Bildgebungsvorrichtung als weitere Bildgebungsvorrichtung eine PET-Vorrichtung, ist bevorzugterweise die Erfassungseinheit zu einer Erfassung einer Position in drei Richtungen des Patiententischs ausgebildet. Die zwei Richtungen oder die drei Richtungen sind dabei orthogonal zueinander ausgerichtet. Mittels der Positionskodiereinheit, die bevorzugt eine zweidimensionale Kodierstruktur umfasst, kann somit eine Positionsinformation in zwei Richtungen des Patiententischs für eine Erfassung durch Erfassungseinheit bereitgestellt werden. Bevorzugt wird mittels der Positionskodiereinheit eine Positionsinformation in Richtung einer Einfahrbewegung in den Patientenaufnahmebereich, insbesondere in die z-Richtung, und eine weitere Richtung, beispielsweise die x-Richtung, des Patiententischs bereitgestellt. Die Positionsinformation, die nicht durch die Positionskodiereinheit bereitgestellt wird, wird mittels des Lasers bereitgestellt und von der Erfassungseinheit erfasst. Werden durch die Positionskodiereinheit Positionsinformationen in z-Richtung und in x-Richtung bereitgestellt, wird mittels des zumindest einen Laser eine Positionsinformation in y-Richtung bereitgestellt. Werden dagegen durch die Positionskodiereinheit Positionsinformationen in z-Richtung und in y-Richtung bereitgestellt, wird mittels des zumindest einen Laser eine Positionsinformation in x-Richtung bereitgestellt. Derart kann mit einer einzigen Erfassungseinheit eine Position des Patiententischs in zwei Richtungen, bevorzugt in alle drei Richtungen, erfasst werden. Dies ermöglicht eine besonders kompakte und effiziente Positionsbestimmungseinheit. Des Weiteren kann für eine Berechnung einer Schwächungskorrektur bei einer PET-Untersuchung eine exakte Position des Patiententischs von der Positionsbestimmungseinheit bereitgestellt werden. Insbesondere können derart abrupte Positionsänderungen und/oder Bewegungen des Patienten auf dem Patiententisch, die zu einer Positionsänderung des Patiententischs in mm-Bereich führen können, schnell und zuverlässig erfasst werden.

In einer vorteilhaften Weiterbildung der erfindungsgemäßen kombinierten Bildgebungsvorrichtung kann es vorgesehen sein, dass die Positionsbestimmungseinheit zumindest eine Erfassungseinheit aufweist, wobei die zumindest eine Erfassungseinheit direkt an eine Field of View (FOV) der weiteren Bildgebungsvorrichtung angrenzend an der den Patientenaufnahmebereich umgebenden Umhausung angeordnet ist. Insbesondere bei einer Ausbildung der weiteren Bildgebungsvorrichtung als PET-Vorrichtung ist eine exakte Bestimmung einer Position des Patiententischs, bevorzugt in alle drei Richtungen, innerhalb des FOVs der PET-Vorrichtung wünschenswert, um eine möglichst exakte Bestimmung einer Schwächungskorrekturkarte zu ermöglichen. Daher ist auch eine Positionierung der zumindest einen Erfassungseinheiten möglichst nah am FOV der PET-Vorrichtung von Vorteil. Weist die Positionsbestimmungseinheit zwei Erfassungseinheiten auf, ist bevorzugt eine erste der Erfassungseinheiten direkt vor dem FOV der PET-Vorrichtung angrenzend an diesem angeordnet und eine zweite der Erfassungseinheiten direkt nach dem FOV angrenzend an diesem angeordnet. Dies ermöglicht es, anhand der Positionen des Patiententischs direkt vor dem FOV der PET-Vorrichtung und direkt nach dem FOV der PET-Vorrichtung auf eine Position des Patiententischs innerhalb des FOV der PET-Vorrichtung zu schließen und diese Position dann als Grundlage für die Berechnung der Schwächungskorrektur zu verwenden. Ein weiterer Vorteil ist, dass mittels der zumindest einen Erfassungseinheit zudem in z-Richtung eine redundante Erfassung von Positionsinformationen des Patiententischs erfolgen kann, wobei eine primäre Erfassung der z-Position des Patiententischs durch einen preiswerten Seilzugencoder erfolgen kann.

In einer vorteilhaften Weiterbildung der erfindungsgemäßen kombinierten Bildgebungsvorrichtung kann es vorgesehen sein, dass die Positionsbestimmungseinheit zumindest zwei Erfassungseinheiten aufweist, wobei eine der Erfassungseinheiten in einem Eingangsbereich des Patientenaufnahmebereichs an der den Patientenaufnahmebereich umgebenden Umhausung angeordnet ist. Derart kann direkt bei einem Einfahren des Patiententischs in den Patientenaufnahmebereich bereits eine Positionsinformation zur Bestimmung der Position des Patiententischs bestimmt werden. Insbesondere ermöglicht diese Position bereits eine exakte Bestimmung in die Bewegungsrichtung des Patiententischs, insbesondere in die z-Richtung. Für eine Erfassung der Position des Patiententischs in eine weitere Richtung, insbesondere in x-Richtung oder in die y-Richtung, wird die zweite der Erfassungseinheiten bevorzugt in unmittelbarer Nähe zum FOV an der den Patientenaufnahmebereich umgebenden Umhausung angeordnet. So kann zudem in z-Richtung eine redundante Erfassung von Positionsinformationen des Patiententischs erfolgen.

Weitere Vorteile, Merkmale und Einzelheiten der Erfindung ergeben sich aus dem im Folgenden beschriebenen Ausführungsbeispiel sowie anhand der Zeichnungen.

Es zeigen:
- Fig. 1: eine erfindungsgemäße kombinierte Bildgebungsvorrichtung mit einer Patientenlagerungsvorrichtung und einer Positionsbestimmungseinheit in einer schematischen Seitenansicht,
- Fig. 2: die kombinierte Bildgebungsvorrichtung in einer Frontansicht,
- Fig. 3: eine Erfassungseinheit der Positionsbestimmungseinheit bei der Erfassung einer Positionsinformation in z-Richtung und in x-Richtung,
- Fig. 4: die Erfassungseinheit der Positionsbestimmungseinheit bei der Erfassung einer Positionsinformation in y-Richtung,
- Fig. 5: ein erstes Ausführungsbeispiel einer Kodierung der Positionsbestimmungseinheit und,
- Fig. 6: ein zweites Ausführungsbeispiel einer Kodierung der Positionsbestimmungseinheit.

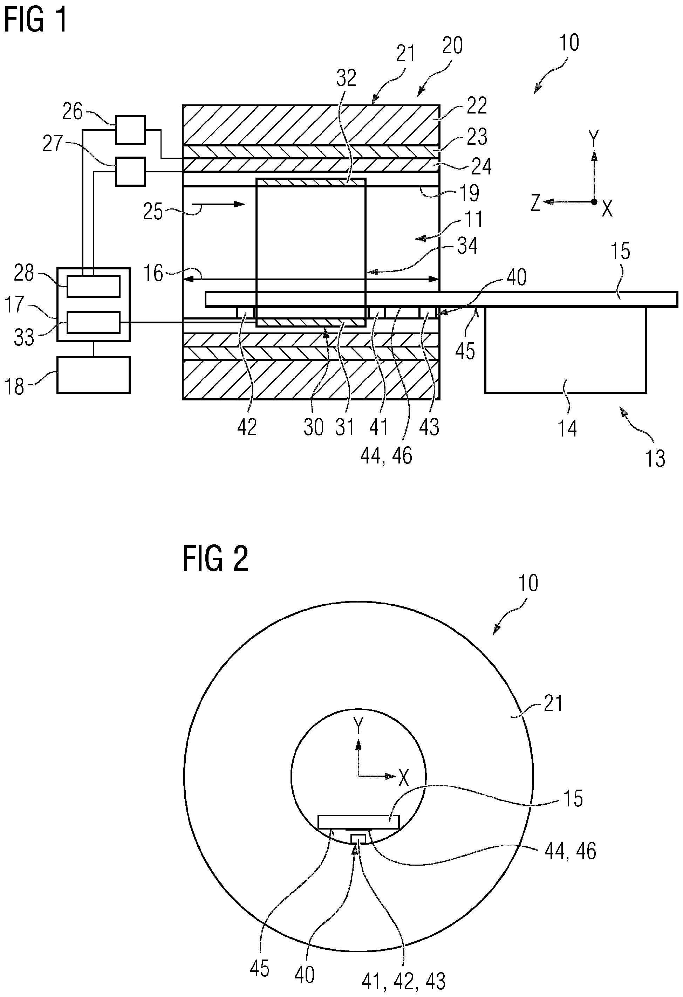

In der Fig. 1 ist eine kombinierte Bildgebungsvorrichtung 10 schematisch dargestellt. Die kombinierte Bildgebungsvorrichtung 10 weist eine erste medizinische Bildgebungsvorrichtung auf, die als Magnetresonanzvorrichtung 20 ausgebildet ist. Die kombinierte Bildgebungsvorrichtung 10 weist eine weitere Bildgebungsvorrichtung 30 auf, die im vorliegenden Ausführungsbeispiel als PET-Vorrichtung ausgebildet ist. Die vorliegende Erfindung ist jedoch nicht auf Ausgestaltung der zweiten medizinischen Bildgebungsvorrichtung 30 auf eine PET-Vorrichtung beschränkt und weitere Ausgestaltungen der weiteren medizinischen Bildgebungsvorrichtung 30 sind jederzeit denkbar, wie beispielsweise eine Ausgestaltung als Röntgenvorrichtung usw.

Die Magnetresonanzvorrichtung 20 weist eine als Magneteinheit 21 ausgebildete Scannereinheit auf. Die Magneteinheit 21 umfasst einen Grundmagneten 22, eine Gradientenspuleneinheit 23 und eine Hochfrequenzantenneneinheit 24. Der Grundmagnet 22 der Magneteinheit 21 ist zu einem Erzeugen eines starken und insbesondere konstanten Grundmagnetfelds 25 ausgebildet. Dabei kann der Grundmagnet 22 beispielsweise als supraleitender Grundmagnet 22 oder auch als Dauermagnet ausgebildet sein. Die Gradientenspuleneinheit 23 der Magneteinheit 21 ist zu einer Erzeugung von Magnetfeldgradienten, die für eine Ortskodierung während einer Bildgebung verwendet werden, ausgebildet. Die Gradientenspuleneinheit 23 wird mittels einer Gradientensteuereinheit 26 der Magnetresonanzvorrichtung 20 gesteuert. Die Hochfrequenzantenneneinheit 24 der Magneteinheit 21 ist zu einer Anregung einer Polarisation, die sich in dem von dem Grundmagneten 22 erzeugten Grundmagnetfeld 25 einstellt, ausgebildet. Die Hochfrequenzantenneneinheit 24 wird von einer Hochfrequenzantennensteuereinheit 27 der Magnetresonanzvorrichtung 20 gesteuert und strahlt hochfrequente Magnetresonanzsequenzen in einen Patientenaufnahmebereich 11 ein.

Zu einer Steuerung des Grundmagneten 22, der Gradientensteuereinheit 26 und zur Steuerung der Hochfrequenzantennensteuereinheit 27 weist die Magnetresonanzvorrichtung 20 eine Magnetresonanz-Steuereinheit 28 auf. Die Magnetresonanz-Steuereinheit 28 steuert zentral die Magnetresonanzvorrichtung 20, wie beispielsweise das Durchführen einer vorbestimmten bildgebenden Gradientenechosequenz. Zudem umfasst die Magnetresonanz-Steuereinheit 28 eine nicht näher dargestellte Auswerteeinheit zu einer Auswertung von medizinischen Magnetresonanzbilddaten.

Die dargestellte Magnetresonanzvorrichtung 20 kann selbstverständlich weitere Komponenten umfassen, die Magnetresonanzvorrichtungen 20 gewöhnlich aufweisen. Eine allgemeine Funktionsweise einer Magnetresonanzvorrichtung 20 ist zudem dem Fachmann bekannt, so dass auf eine detaillierte Beschreibung der allgemeinen Komponenten verzichtet wird.

Die PET-Vorrichtung umfasst eine Scannereinheit 31 mit mehreren Positronen-Emissions-Tomographie-Detektormodulen 32 (PET-Detektormodule 32), die zu einer Ringform angeordnet sind und den Patientenaufnahmebereich 11 in der Umfangsrichtung umgeben. Die PET-Detektormodule 31 weisen jeweils mehrere, nicht näher dargestellte Positronen-Emissions-Tomographie-Detektorelemente (PET-Detektorelemente) auf, die zu einem PET-Detektorarray angeordnet sind, das ein Szintillationsdetektorarray mit Szintillationskristallen, beispielsweise LSO-Kristalle, umfasst. Des Weiteren umfassen die PET-Detektormodule jeweils ein Photodiodenarray, beispielsweise Avalanche-Photodiodenarray oder APD-Photodiodenarray, die dem Szintillationsdetektorarray nachgeschaltet innerhalb der PET-Detektormodule 32 angeordnet sind.

Mittels der PET-Detektormodule 32 werden Photonenpaare, die aus der Annihilation eines Positrons mit einem Elektron resultieren, erfasst. Trajektorien der beiden Photonen schließen einen Winkel von 180° ein. Zudem weisen die beiden Photonen jeweils eine Energie von 511 keV auf. Das Positron wird hierbei von einem Radiopharmakon emittiert, wobei das Radiopharmakon über eine Injektion dem Patienten verabreicht wird. Beim Durchlaufen von Materie im Strahlengang können die bei der Annihilation entstandenen PET-Photonen abgeschwächt werden, wobei die Abschwächungswahrscheinlichkeit von der Pfadlänge durch die Materie und dem entsprechenden Abschwächungskoeffizienten der Materie abhängt.

Zudem weisen die PET-Detektormodule 32 jeweils eine nicht näher dargestellte Detektorelektronik auf, die eine elektrische Verstärkerschaltung und weitere, nicht näher dargestellte Elektronikkomponenten umfasst.

Zu einer Steuerung der Detektorelektronik und der PET-Detektormodule 32 weist die PET-Vorrichtung eine PET-Steuereinheit 33 auf. Die PET-Steuereinheit 33 steuert zentral die PET-Vorrichtung. Zudem umfasst die PET-Steuereinheit 33 eine Auswerteeinheit zu einer Auswertung von erfassten PET-Daten auf.

Die dargestellte PET-Vorrichtung kann selbstverständlich weitere Komponenten umfassen, die PET-Vorrichtungen gewöhnlich aufweisen. Eine allgemeine Funktionsweise einer PET-Vorrichtung ist zudem dem Fachmann bekannt, so dass auf eine detaillierte Beschreibung der allgemeinen Komponenten verzichtet wird.

Die beiden medizinischen Bildgebungsvorrichtungen 30 sind im vorliegenden Ausführungsbeispiel derart ausgebildet, dass die PET-Scannereinheit 31 der zweiten medizinischen Bildgebungsvorrichtung 30 in die Scannereinheit, insbesondere die Magneteinheit 21, der Magnetresonanzvorrichtung 20 integriert ist. Somit steht nur ein einziges Gerät mit einem einzigen Patientenaufnahmebereich 11 für eine Untersuchung des Patienten, insbesondere der zu untersuchenden Bereichs des Patienten, zur Verfügung.

Die kombinierte Bildgebungsvorrichtung 10 weist den Patientenaufnahmebereich 11 auf zu einer Aufnahme einer kombinierte MR-PET-Untersuchung an dem Patienten. Der Patientenaufnahmebereich 11 im vorliegenden Ausführungsbeispiel ist zylinderförmig ausgebildet und in einer Umfangsrichtung von der Magneteinheit 21 und der Scannereinheit 31 der PET-Vorrichtung zylinderförmig umgeben. Grundsätzlich ist jedoch eine davon abweichende Ausbildung des Patientenaufnahmebereichs 11 jederzeit denkbar. Die kombinierte Bildgebungsvorrichtung 10 weist dabei eine den Patientenaufnahmebereich 11 zylinderförmig umgebenden Umhausung 19 auf.

Für eine Positionierung des Patienten, insbesondere eines zu untersuchenden Bereichs des Patienten, innerhalb des Patientenaufnahmebereichs 11 weist die kombinierte Bildgebungsvorrichtung 10 eine Patientenlagerungsvorrichtung 13 auf. Die Patientenlagerungsvorrichtung 13 weist eine Basiseinheit 14 und einen bezüglich der Basiseinheit 14 bewegbaren Patiententisch 15 auf. Der Patiententisch 15 ist für eine Positionierung des Patienten, insbesondere des zu untersuchenden Bereichs des Patienten, bewegbar innerhalb des Patientenaufnahmebereichs 11 ausgebildet. Insbesondere ist hierbei der Patiententisch 15 in Richtung einer Längserstreckung 16 des Patientenaufnahmebereichs 11 und/oder in z-Richtung bewegbar gelagert.

Die kombinierte Bildgebungsvorrichtung 10, insbesondere die MR-PET-Vorrichtung, weist zudem eine zentrale Recheneinheit 17 auf, die beispielsweise eine Erfassung und/oder eine Auswertung von Magnetresonanzsignalen und von PET-Signalen aufeinander abstimmt. Die Recheneinheit 17 kann eine zentrale Systemsteuereinheit sein.

Des Weiteren umfasst die kombinierte Bildgebungsvorrichtung 10, insbesondere die MR-PET-Vorrichtung, eine Benutzerschnittstelle 18, die mit der zentralen Recheneinheit 17 verbunden ist. Steuerinformationen wie beispielsweise Bilddaten können auf einer nicht näher dargestellten Ausgabeeinheit, beispielsweise auf zumindest einem Monitor, der Benutzerschnittstelle 18 für ein medizinisches Bedienpersonal angezeigt werden. Weiterhin weist die Benutzerschnittstelle 18 eine nicht näher dargestellte Eingabeeinheit auf, mittels der Informationen und/oder Parameter während eines Messvorgangs von dem medizinischen Bedienpersonal eingegeben werden können.

Für eine PET-Untersuchung ist es üblich, eine Schwächungskorrekturkarte zur Verfügung zu stellen, um einen Energieverlust der Photonen bei der Erfassung der PET-Ereignisse, insbesondere der Photonen, zu berücksichtigen. Hierzu ist exakte Erfassung einer Position des Patiententischs 15 erforderlich, wobei der Patiententischs in die Bestimmung einer Schwächungskorrektur und/oder der Schwächungskorrekturkarte eingeht. Die kombinierte Bildgebungsvorrichtung 10 weist hierzu eine Positionsbestimmungseinheit 40 auf, die zu einer Bestimmung einer Position des Patiententischs 15 in zumindest eine Richtung ausgebildet ist. Im vorliegendem Ausführungsbeispiel ist die Positionsbestimmungseinheit 40 zu einer Bestimmung der Position des Patiententischs in drei Richtung, insbesondere in z-Richtung, in x-Richtung und in y-Richtung, ausgebildet. Die drei Richtungen, insbesondere die z-Richtung, die y-Richtung und die x-Richtung sind dabei orthogonal zueinander ausgerichtet. Grundsätzlich kann in einer alternativen Ausgestaltung der Positionsbestimmungseinheit 40 diese auch nur zur Bestimmung der Position des Patiententischs 14 in eine einzige Richtung, bevorzugt in z-Richtung, oder auch in zwei Richtungen, bevorzugt in z-Richtung und eine weitere Richtung, ausgebildet sein.

Zur Bestimmung der Position des Patiententischs 15 weist die Positionsbestimmungseinheit 40 zumindest eine Erfassungseinheit 41, 42, 43 und eine Positionskodiereinheit 44 auf. Im vorliegenden Ausführungsbeispiel weist die Positionsbestimmungseinheit 40 drei Erfassungseinheiten 41, 42, 43 auf (Fig. 1). In einer alternativen Ausgestaltung der Positionsbestimmungseinheit 40 kann diese auch nur eine einzige Erfassungseinheit 41 oder auch zwei Erfassungseinheiten 41, 42 aufweisen. Die einzelnen Erfassungseinheiten 41, 42, 43 sind an der den Patientenaufnahmebereich 11 umgebenden Umhausung 19 angeordnet. Die Erfassungseinheiten 41, 42, 43 sind dazu ausgebildet, eine Positionsinformation der Positionskodiereinheit 44 in zumindest eine Richtung zu erfassen. Im vorliegenden Ausführungsbeispiel sind die Erfassungseinheiten 41, 42, 43 zu einer Erfassung einer Positionsinformation der Positionskodiereinheit 44 in alle drei Richtungen, insbesondere in x-Richtung, in y-Richtung und in z-Richtung, ausgebildet.

Die Positionskodiereinheit 44 dagegen ist am Patiententisch 15 angeordnet. Dabei ist die Positionskodiereinheit 44 an einer Seite des Patiententischs 15 angeordnet, die keine zur Lagerung des Patienten ausgebildete Fläche umfasst. Im vorliegenden Ausführungsbeispiel ist die Positionskodiereinheit 44 an einer Unterseite 45 des Patiententischs 15 angeordnet. Die Positionskodiereinheit 44 umfasst ein Kodierband 46, das an der Unterseite 45 des Patiententischs 15 angeordnet ist. Bevorzugt ist das Kodierband an die Unterseite 45 des Patiententischs 15 angeklebt. Das Kodierband 46 erstreckt sich dabei über die gesamte Länge des Patiententischs 15, so dass das Kodierband 46 in z-Richtung eine Länge umfasst, die einer Länge des Patiententischs 15 entspricht. Beispielsweise weist das Kodierband 46 eine Länge von 2.200 mm auf. Zudem kann das Kodierband 46 eine Breite von beispielsweise 30 mm und eine Dicke von beispielsweise 3 mm umfassen. Das Kodierband 46 kann dabei beispielsweise ein Kunststoffband mit einem PVC-Material und/oder weiteren, dem Fachmann als sinnvoll erscheinende Materialien umfassen.

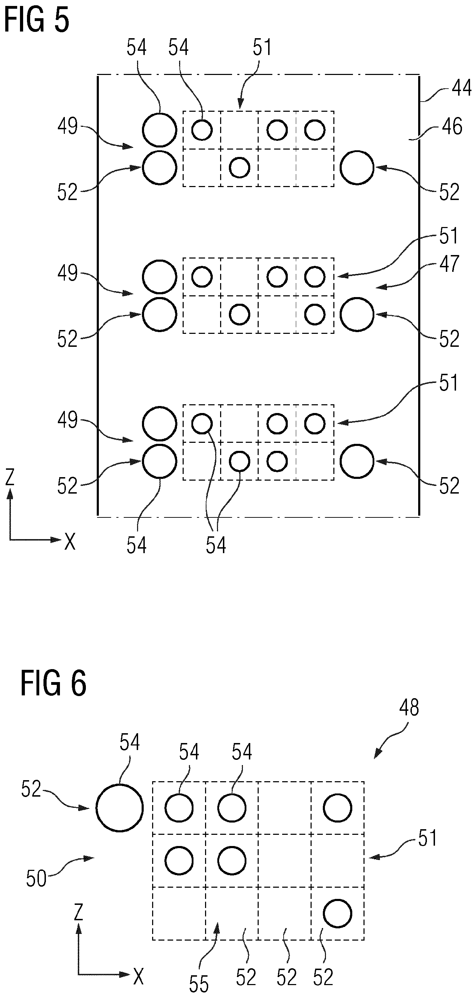

Die Positionskodiereinheit 44 weist eine Kodierung 47, 48 mit mehreren Kodierstrukturen 49, 50 auf (Fig. 5 und 6), wobei die einzelnen Kodierstrukturen 49, 50 jeweils eine Positionsinformation in z-Richtung des Patiententischs und eine Positionsinformation in x-Richtung des Patiententischs 15 umfassen. Die einzelnen Kodierstrukturen 49, 50 umfassen dabei jeweils eine Positionskodierung 51, die einem definierten Längenabschnitt am Patiententisch 15 zugeordnet ist, und eine Informationskodierung 52, die zusätzliche Information zur Positionskodierung 51 bereitstellt (Fig. 5 und 6). Die Positionskodierung 51 weist eine zweidimensionale Struktur auf dem Kodierband 46 auf, so dass in diese zwei Dimensionen, im vorliegenden Ausführungsbeispiel die z-Richtung und die x-Richtung, eine Positionsinformation für eine Erfassung durch die Erfassungseinheiten 41, 42, 43 bereitgestellt wird.

Die Länge des Kodierbands 46 ist dabei in mehrere Längenabschnitte unterteilt, wobei jeder der mehreren Längenabschnitte mittels einer der mehreren Kodierstrukturen 49, 50, insbesondere der Positionskodierungen 51, der Positionskodiereinheit 44 eindeutig definiert ist und durch eine Erfassung der Positionskodierung 51 identifiziert werden kann. Die einzelnen Längenabschnitte des Patiententischs 15, insbesondere der Länge des Patiententischs 15 in z-Richtung, weisen eine maximale Größe von 20 mm auf. Bevorzugt weist ein Längenabschnitt des Patiententischs 15, insbesondere der Länge des Patiententischs 15 in z-Richtung, maximal 18 mm auf. Bevorzugt weist ein Längenabschnitt des Patiententischs 15, insbesondere der Länge des Patiententischs 15 in z-Richtung 15, maximal 16 mm auf. Bevorzugt weist ein Längenabschnitt des Patiententischs 15, insbesondere der Länge des Patiententischs 15 in z-Richtung, maximal 14 mm auf. Bevorzugt weist ein Längenabschnitt des Patiententischs 15, insbesondere der Länge des Patiententischs 15 in z-Richtung, maximal 12 mm auf. Bevorzugt weist ein Längenabschnitt des Patiententischs 15, insbesondere der Länge des Patiententischs 15 in z-Richtung, maximal 10 mm auf. Bevorzugt weist ein Längenabschnitt des Patiententischs 15, insbesondere der Länge des Patiententischs 15 in z-Richtung, maximal 9 mm auf. Bevorzugt weist ein Längenabschnitt des Patiententischs 15, insbesondere der Länge des Patiententischs 15 in z-Richtung, maximal 8 mm auf. Bevorzugt weist ein Längenabschnitt des Patiententischs 15, insbesondere der Länge des Patiententischs 15 in z-Richtung, maximal 7 mm auf. Bevorzugt weist ein Längenabschnitt des Patiententischs 15, insbesondere der Länge des Patiententischs 15 in z-Richtung, maximal 6 mm auf. Bevorzugt weist ein Längenabschnitt des Patiententischs 15, insbesondere der Länge des Patiententischs 15 in z-Richtung, maximal 5 mm auf.

Die Kodierung 47, 48, insbesondere die einzelnen Kodierstrukturen 49, 50, weisen jeweils einen Binärcode auf. Der Binärcode kann dabei eine 8-Bit-Kodierung mit einer 2x4-Codematrix umfassen, wie dies in Fig. 5 dargestellt ist. Dabei umfasst die 2x4-Codematrix eine Matrix mit zwei Reihen und vier Spalten, in der der Binärcode auf die zwei Reihen aufgeteilt ist. Der Binärcode, insbesondere die 8-Bit-Kodierung, umfasst dabei die Positionskodierung 51 der Kodierung 47, insbesondere der einzelnen Kodierstrukturen 49. Der 8-Bit-Kodierung stehen dabei 255 unterschiedliche Positionskodierungen 51 zur Einteilung der Länge des Patiententischs 15 in Längenabschnitte zur Verfügung. Beispielsweise umfasst hierbei eine Kodierstruktur 49 einen Längenabschnitt des Patiententischs 15 von 10 mm Länge. Dabei umfasst der Binärcode 00000001 den ersten Längenabschnitt mit dem ersten cm der Länge des Patiententischs 15. Der Binärcode 01100100 umfasst den 100. Längenabschnitt an der Position 100 cm der Länge des Patiententischs 15.

Zusätzlich weist die Kodierung 47, insbesondere die einzelnen Kodierstrukturen 49, eine Informationskodierung 52 auf, wobei die Informationskodierung 52 einen Richtungscode und/oder einen Markierungscode umfasst.

Die Kodierung 47, insbesondere der Binärcode ist im vorliegenden Ausführungsbeispiel in das Kodierband 46 eingefräst und/oder eingebohrt, indem Ausnehmungen 54 in Form von Kreisflächen in das Kodierband 46 eingefräst und/oder eingebohrt sind. Eine derartige Ausnehmung 54 in Form einer Kreisfläche kann beispielsweise einen Durchmesser 1 mm oder 2 mm aufweisen. Diese Ausnehmungen 54 sind zur besseren Sichtbarkeit, insbesondere eines besseren Kontrasts gegenüber dem Kodierband 46, und/oder zu besseren Erfassung mittels der Erfassungseinheiten 41, 42, 43 zusätzlich mit einer Farbe gefüllt. Bevorzugt weist die Farbe der Füllung der Ausnehmungen 54 einen hohen farblichen Kontrast zu einer Farbe des Kodierbands 46 auf. Im vorliegendem Ausführungsbeispiel weist das Kodierband 46 eine weiße Farbe auf und die Ausnehmungen 54 eine schwarze Farbe auf. Die Farbe zum Befüllen der Ausnehmungen 54 kann beispielsweise ein 2-Komponenten Epoxidharz mit schwarzen Pigmenten umfassen. Zudem ist es auch denkbar, für unterschiedliche Ausnehmungen 54 Füllungen unterschiedliche Farben zu verwenden.

Im vorliegenden Ausführungsbeispiel (Fig. 5) weisen die Ausnehmungen 54, die von der Positionskodierung 51 umfasst sind, einen Durchmesser auf, der halb so groß ist wie ein Durchmesser der Ausnehmungen 54, die von der Informationskodierung 52 umfasst sind. Somit kann eine Verwechslung der Kodierung 47, insbesondere der Positionskodierung 51 und der Informationskodierung 52, bei einer Erfassung der Kodierung 47 durch die Erfassungseinheiten 41, 42, 43 und einer Bestimmung der Position des Patiententischs 15 vorteilhaft verhindert werden.

Die Informationskodierung 52, insbesondere der Richtungscode und/oder der Markierungscode, ist dabei links und rechts neben der Positionskodierung 51, insbesondere der 8-Bit-Kodierung mit der 2x4-Codematrix, angeordnet und markiert den Bereich, der für die Positionskodierung 51, insbesondere die 8-Bit-Kodierung mit der 2x4-Codematrix, zur Verfügung steht. Der Richtungscode und/oder Markierungskode links neben der Positionskodierung 51 und der Richtungscode und/oder Markierungskode rechts neben der Positionskodierung 51 sind unterschiedlich ausgebildet. Im vorliegenden Ausführungsbeispiel sind links neben der Positionskodierung 51 zwei Markierungspunkte des Richtungscodes und/oder des Markierungscodes und rechts neben der Positionskodierung 51 ein Markierungspunkt des Richtungscodes und/oder des Markierungscodes angeordnet (Fig. 5).

In Fig. 5 ist ein Abschnitt des Kodierbands 46 mit drei nacheinander folgenden Kodierstrukturen 49 dargestellt. Die drei Kodierstrukturen 49 weisen jeweils die gleiche Informationskodierung 52 insbesondere den gleichen Richtungscode und/oder Markierungscode auf. Dabei sind links neben der Positionskodierung 51, insbesondere der 8-Bit-Kodierung mit der 2x4-Codematrix, zwei Ausnehmungen 54 der Informationskodierung 52 und rechts neben der Positionskodierung 51, insbesondere der 8-Bit-Kodierung mit der 2x4-Codematrix, eine Ausnehmung 54 der Informationskodierung 52 angeordnet. Die drei Kodierstrukturen 49 weisen dabei eine fortlaufende Positionskodierung 51 auf. Dabei weist die erste Kodierstruktur 49, in Fig. 5 die obere Kodierstruktur 49, eine Positionskodierung 51 mit dem Wert 180 auf. Die zweite Kodierstruktur 49 in Fig. 5 die mittlere Kodierstruktur 49, weist eine Positionskodierung 51 mit dem Wert 181 auf. Die dritte Kodierstruktur 49, in Fig. 5 die untere Kodierstruktur 49, weist eine Positionskodierung 51 mit dem Wert 182 auf. Eine Mitte der Positionskodierung 51, insbesondere der 2x4-Codematrix definiert dabei den entsprechenden Längenabschnitt. Im vorliegenden Ausführungsbeispiel entspricht die obere Kodierstruktur 49, insbesondere eine Mitte der oberen Kodierstruktur 49, einer Länge von 180 cm des Patiententischs 15, die mittlere Kodierstruktur 49, insbesondere eine Mitte der mittleren Kodierstruktur 49, einer Länge von 181 cm des Patiententischs 15 und die untere Kodierstruktur 49, insbesondere eine Mitte der unteren Kodierstruktur 49, einer Länge von 182 cm des Patiententischs 15.

In Fig. 6 ist ein zu Fig. 5 alternatives Ausführungsbeispiel für eine Kodierung 48 dargestellt. Die Kodierung 48, insbesondere die einzelnen Kodierstrukturen 50, weisen jeweils ebenfalls einen Binärcode auf. Der Binärcode umfasst dabei eine 9-Bit-Kodierung mit einer 3x4-Codematrix. Der Binärcode, insbesondere die 9-Bit-Kodierung, umfasst dabei die Positionskodierung 51 der Kodierung 48, insbesondere der einzelnen Kodierstrukturen 50. Der 9-Bit-Kodierung stehen dabei 511 unterschiedliche Positionskodierungen 51 zur Einteilung der Länge des Patiententischs 15 in Längenabschnitte zur Verfügung. Hierbei umfasst eine Kodierstruktur 49 einen Längenabschnitt des Patiententischs 15 von 5 mm Länge. Die 3x4-Codematrix umfasst eine Matrix mit drei Reihen und vier Spalten, in der der Binärcode auf die drei Reihen aufgeteilt ist, wobei die letzte Spalte mit nur einem Bit für die 9-Bit-Kodierung, insbesondere die Positionskodierung 51, versehen ist. Die restlichen drei Bits stehen zumindest teilweise für die Informationskodierung 52 der Kodierung 48 zur Verfügung. Diese umfasst im vorliegenden Ausführungsbeispiel (Fig. 6) einen Markierungscode und einen Paritätscode 55. Zudem bleibt ein Platz der 3×4-Code-matrix frei. Zudem weist im vorliegendem Ausführungsbeispiel die Informationskodierung 52 der Kodierung 48, insbesondere die einzelnen Kodierstrukturen 49, einen Markierungscode auf, der links neben der Positionskodierung 51 angeordnet ist und der einen doppelten Durchmesser aufweist, als die einzelnen Kodierungs-Bits des Binärcodes.

Alternativ zu einer 3x4-Codematrix kann die Kodierung 48, insbesondere die Positionskodierung 51, bei einer 9-Bit-Kodierung auch eine 3x3-Codematrix umfassen.

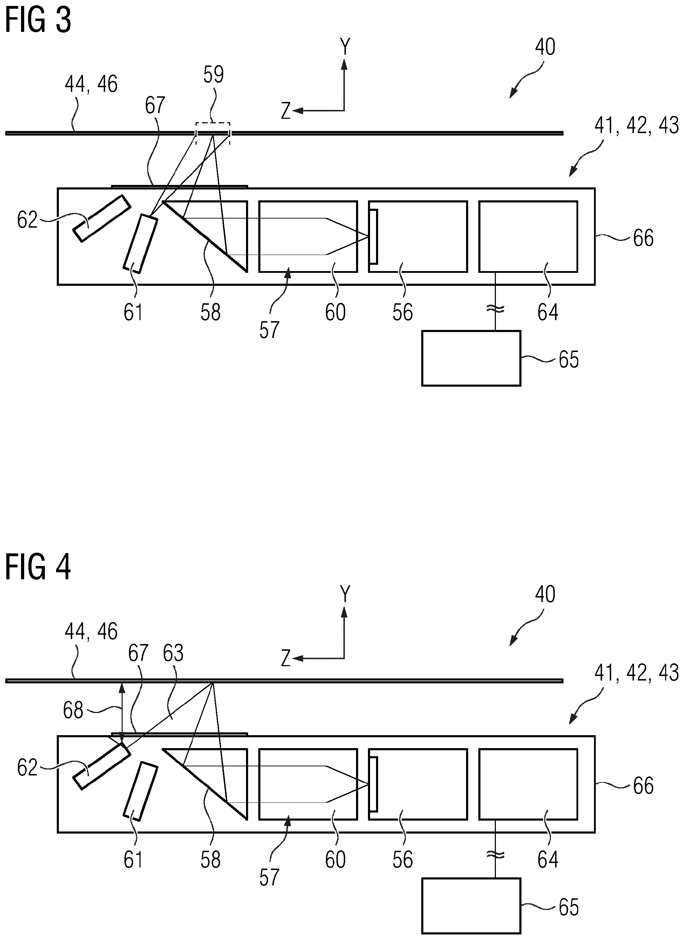

Zur Erfassung der Kodierung 47, 48 weist im vorliegendem Ausführungsbeispiel die Positionsbestimmungseinheit 40 drei Erfassungseinheiten 41, 42, 43 auf (Fig. 1), wobei ein Aufbau und eine Funktionsweise anhand einer Erfassungseinheit 41, 42, 43 im Folgendem (Fig. 3 und 4) näher erläutert wird. Die Erfassungseinheit 41, 42, 43 weist eine Kamera 56 auf. Die Kamera 56 weist dabei eine Auflösung von mindestens 400 Pixeln in Erfassungsrichtung auf. Beispielsweise kann die Kamera eine VGA-Kamera mit einer Auflösung von 640x480 Pixeln in die jeweilige Erfassungsrichtung aufweisen. Dabei kann die Kamera 56 einen CCD-/CMOS-Sensor umfassen. Umfasst beispielsweise die Kamera 56 einen Erfassungsbereich 59 oder ein FOV von 1x1 cm, können die Kodierstrukturen 49, 50 mit einer Auflösung von 0,015 mm in z-Richtung von der Kamera 56 erfasst werden. Da die Kodierung 47, 48, insbesondere die einzelnen Kodierstrukturen 49, 50, eine zweidimensionale Struktur aufweisen, können bei einem derartigen Erfassungsbereich 59 der Kamera 56 in x-Richtung die Kodierstrukturen 49, 50 mit einer Auflösung von 0,021 mm von der Kamera 56 erfasst werden. Zudem können auch Kameras mit einer höheren Auflösung, beispielsweise von 1000 Pixeln in jede Erfassungsrichtung, verwendet werden.

Die Erfassungseinheit 41, 42, 43 weist weiterhin eine Erfassungsoptik 57 mit einem Prisma 58 auf, wobei die Erfassungsoptik 57 der Kamera 56 vorgeschaltet angeordnet ist. Das Prisma 58 umfasst dabei ein 45°-Prisma, das einen Strahlengang der Erfassungseinheit 41, 42, 43, insbesondere der Kamera 56, um 90° bricht. Dies ermöglicht eine besonders platzsparende Anordnung, da die Kamera 56, insbesondere eine Erfassungsrichtung der Kamera 56, somit parallel zur Längserstreckung 16 des Patiententischs 15 und damit parallel zum Kodierband 46 ausgerichtet ist. Durch das Prisma 58 wird der Erfassungsbereich 59 der Kamera 56 auf das Kodierband 46 gelenkt. Die Erfassungsoptik 57 umfasst zudem ein Objektiv 60, das zwischen der Kamera 56 und dem Prisma 58 angeordnet ist.

Die Erfassungseinheit 41, 42, 43 weist weiterhin zumindest eine LED 61 auf (Fig. 3 und 4). Dabei kann die Erfassungseinheit 41, 42, 43 auch mehrere LEDs 61 aufweisen. Die zumindest eine LED 61 ist zu einer Ausleuchtung des Erfassungsbereichs 59, insbesondere des von der Kamera 56 erfassten Bereichs des Kodierbands 46 an der Unterseite 45 des Patiententischs 15, ausgebildet. Dabei ist eine Intensität und eine Lichtfarbe eines von der zumindest einen LED 61 ausgesendeten Lichts auf eine Farbe des Kodierbands 46 und eine Farbe der Ausnehmungen 54 der Kodierung 47, 48 abgestimmt, um einen maximalen Kontrast zwischen den Ausnehmungen 54 und dem Kodierband 46 bei der Erfassung der Kodierung 47, 48 zu erzielen.

Des Weiteren weist die Erfassungseinheit 41, 42, 43 einen Laser 62 auf (Fig. 3 und 4). Der Laser 62 ist zu einem Bereitstellen einer Positionsinformation in eine dritte Richtung, insbesondere die y-Richtung, des Patiententischs 15 ausgebildet. Der Laser 62 generiert zum Bereitstellen der Positionsinformation in y-Richtung einen Laserstrahl 63, der auf den Patiententisch 15, insbesondere auf die Unterseite 45 des Patiententischs 15, gerichtet ist. Dabei ist der Laserstrahl 63 mit einem von 90° abweichenden Winkel, beispielsweise einem Winkel zwischen 30° und 60°, auf den Patiententisch 15, insbesondere die Unterseite 45 des Patiententischs 15, gerichtet, so dass der von dem Patiententisch 15 reflektierter Laserstrahl 63 auf das Prisma 58 trifft und von der Kamera 56 erfasst wird.

Die Erfassungseinheit 41, 42, 43 weist zudem eine Elektronikeinheit 64 und eine Steuereinheit 65 auf (Fig. 3 und 4). Die von der Kamera 56 erfassten Positionsdaten werden über die Elektronikeinheit 64 an die Steuereinheit 65 für eine Auswertung übertragen. Die Steuereinheit 65 der Erfassungseinheit 41, 42, 43 ist bevorzugt außerhalb des Patientenaufnahmebereichs 11 angeordnet. Eine Datenübertragung zwischen der Kamera 56 und/oder einer Elektronikeinheit 64 und der Steuereinheit 65 und damit eine Datenübertragung zwischen einem Bereich innerhalb des Patientenaufnahmebereichs 11 und einem Bereich außerhalb des Patientenaufnahmebereichs 11 erfolgt bevorzugt über Kabel. Dabei können die Kabel beispielsweise Lichtwellenleiter und/oder weitere, dem Fachmann als sinnvoll erscheinende Kabel umfassen. Neben einer Auswertung der von der Kamera 56 erfassten Positionsinformationen ist die Steuereinheit 65 zu einer Steuerung der Erfassung der Positionsinformationen in die unterschiedlichen Raumrichtungen mittels der Kamera 56 ausgebildet. Dabei steuert die Steuereinheit 65 sowohl die Kamera 56 als auch den zumindest einen Laser 62 und die zumindest eine LED 61. Insbesondere ist die Steuereinheit 65 dazu ausgebildet, einen Betriebsmodus des zumindest einen Lasers 62 und der zumindest einen LED 61 auf einen Betriebsmodus der die Kamera 56 abzustimmen. Zudem ist die Steuereinheit 65 dazu ausgebildet, die erfasste Position des Patiententischs 15 an die PET-Steuereinheit 33 zu übertragen.

Für eine Anordnung der Kamera 56, der Erfassungsoptik 57, der zumindest einen LED 61, des zumindest einen Lasers 62 und der Elektronikeinheit 64 weist die Erfassungseinheit 41, 42, 43 ein Schirmgehäuse 66 mit einem Sichtfenster 67 auf (Fig. 3 und 4). Das Schirmgehäuse 66 schirmt die Erfassungseinheit 41, 42, 43 bezüglich einer Hochfrequenzstrahlung ab. Dabei ist das Sichtfenster 67 an einer dem Patientenaufnahmebereich 11 und/oder dem Patiententisch 15 zugewandten Seite des Schirmgehäuses 66 angeordnet. Das Sichtfenster 67 umfasst bevorzugt eine transparente Abdeckung, die eine Erfassung von Positionsinformationen mittels der Kamera 56 ermöglicht. Bevorzugt weist das Sichtfenster 67, insbesondere die transparente Abdeckung, ebenfalls bezüglich Hochfrequenzstrahlung abschirmende Eigenschaften auf. Das Sichtfenster 67 umfasst vorteilhaft ein bruchsicheres Glas. Für die abschirmende Eigenschaft weist das Sichtfenster, insbesondere das Glas eine elektrisch leitende und bevorzugt transparente Beschichtung auf. Beispielsweise kann eine derartige Beschichtung des Glases ein ITO-Material umfassen. Alternativ oder zusätzlich ist es auch denkbar, dass die transparente Abdeckung eine Beschichtung, beispielsweise eine dünne leitende Schicht aus Silber, umfasst.

In Fig. 3 ist die Erfassungseinheit 41, 42, 43 in einem Erfassungsmodus zur Erfassung einer Positionsinformation in z-Richtung und in x-Richtung dargestellt. Dabei wird mittels der zumindest einen LED 61 der Patiententisch 15 von unten angeleuchtet und damit auch das an der Unterseite 45 des Patiententischs 15 angeordnete Kodierband 46. Durch das Ausleuchten der Unterseite 45 des Patiententischs 15, insbesondere des an der Unterseite 45 des Patiententischs 15 angeordneten Kodierbands 46, wird dabei ein Kontrast zwischen dem Kodierband 46 und den eingefrästen und/oder eingebohrten Kodierstrukturen 49, 50 erhöht. Mittels der Erfassungseinheit 41, 42, 43, insbesondere der Kamera 56, werden die in dem Erfassungsbereich 59 der Kamera 56 angeordneten Kodierstrukturen 49, 50 erfasst. Die Kamera 56 und eine Auswerteelektronik der Erfassungseinheit 41, 42, 43 sind dabei derart kalibriert, dass eine Mitte des Erfassungsbereichs 59 für die Bestimmung der x-Richtung und der z-Richtung maßgebend ist.

Anhand einer Abweichung einer Mitte einer Kodierstruktur 49 von der Mitte des Erfassungsbereichs 59 kann eine exakte Position des Patiententischs 15 in den erfassten Positionsinformationen von der Steuereinheit 65, insbesondere der Auswerteeinheit der Steuereinheit, bestimmt werden.

In Fig. 4 ist die Erfassungseinheit 41, 42, 43 in einem Erfassungsmodus zur Erfassung einer Positionsinformation in y-Richtung dargestellt. Dabei wird mittels des zumindest einen Lasers 62 ein Laserstrahl 63 auf die Unterseite 45 des Patiententischs 15 gestrahlt und dort reflektiert. Der von der Unterseite 45 des Patiententischs 15 reflektierte Laserstrahl 63 trifft auf die Erfassungsoptik 57 mit dem Prisma 57 und wird von der Kamera 56 erfasst. Der zumindest eine Laser 62 ist derart innerhalb des Schirmgehäuses 66 positioniert, dass der Laserstrahl 63 in einem von 90° abweichenden Winkel auf die Unterseite 45 des Patiententischs 15 trifft, beispielsweise mit einem Auftreffwinkel zwischen 30° und 60°. Abhängig von der y-Position des Patiententischs 15 und damit abhängig von einem Abstand 68 des zumindest einen Lasers 62 zu dem Patiententisch 15 in y-Richtung, wird dabei der Laserstrahl 63 mit unterschiedlichem Abstand zu dem zumindest einen Laser 62 in z-Richtung von dem Patiententisch 15, insbesondere der Unterseite 45 des Patiententischs 15, reflektiert. Je tiefer der Patiententisch 15 angeordnet ist, insbesondere je kleiner ein Abstand zwischen der Erfassungseinheit 41, 42, 43 und dem Patiententisch 15 ist, desto geringer ist ein Strahlweg des Laserstrahls 63 von dem zumindest einen Laser 62 zu der Unterseite 45 des Patiententischs 15 und umgekehrt. Damit ergeben sich je nach y-Position unterschiedliche Auftrefforte des Laserstrahls 63 in der Erfassungseinheit 41, 42, 43, insbesondere der Kamera 56. Diese Differenz kann mittels Triangulation in eine Positionsabweichung des Patiententischs 15 in vertikaler Richtung, insbesondere in y-Richtung, bezüglich eines Referenzpunkts in y-Richtung umgerechnet werden. Der Referenzpunkt in y-Richtung kann beispielsweise eine Position des Patiententischs 15 ohne Beladung umfassen.

Im vorliegenden Ausführungsbeispiel weist die Positionsbestimmungseinheit 40 drei Erfassungseinheiten 41, 42, 43 auf, die an unterschiedlichen Positionen, insbesondere an unterschiedlichen Positionen in z-Richtung, innerhalb des Patientenaufnahmebereichs 11 angeordnet sind. Zwei der Erfassungseinheiten 41, 42 sind direkt neben einem FOV 34 der weiteren Bildgebungsvorrichtung 30, insbesondere der PET-Vorrichtung, an der den Patientenaufnahmebereich 11 umgebenden Umhausung 19 angeordnet (Fig. 1). Dabei ist eine erste Erfassungseinheit 41 direkt vor dem FOV 34 der PET-Vorrichtung an der den Patientenaufnahmebereich 11 umgebenden Umhausung 19 angeordnet. Eine zweite Erfassungseinheit 42 ist direkt nach dem FOV 34 der PET-Vorrichtung an der den Patientenaufnahmebereich 11 umgebenden Umhausung 19 angeordnet. Hierdurch kann eine Position des Patiententischs 15 in alle drei Richtungen, insbesondere die x-Richtung, die y-Richtung und die z-Richtung, direkt vor dem FOV 34 der PET-Vorrichtung und direkt nach dem FOV 34 der PET-Vorrichtung bestimmt werden. Anhand der erfassten Positionsinformationen der ersten Erfassungseinheit 41 und der erfassten Positionsinformationen der zweiten Erfassungseinheit 42 kann von der Steuereinheit 65 eine Position des Patiententischs 15 im FOV 34 der PET-Vorrichtung von der Steuereinheit 65, insbesondere der Auswerteeinheit der Steuereinheit 65, ermittelt werden.

Die dritte Erfassungseinheit 43 ist in einem Eingangsbereich des Patientenaufnahmebereichs 11 an der den Patientenaufnahmebereich 11 umgebenden Umhausung 19 angeordnet, so dass direkt beim Einfahren in den Patientenaufnahmebereich 11 bereits eine Position des Patiententischs 15 in die z-Richtung erfasst und von der Steuereinheit 65, insbesondere der Auswerteinheit der Steuereinheit 65, ermittelt werden kann.

Weist die Positionsbestimmungseinheit 40 nur zwei Erfassungseinheiten 41, 42 auf, sind diese bevorzugt direkt neben dem FOV 34 der weiteren Bildgebungsvorrichtung 30, insbesondere der PET-Vorrichtung, an der den Patientenaufnahmebereich 11 umgebenden Umhausung 19 angeordnet. Bevorzugt ist hierbei die erste Erfassungseinheit 41 direkt vor dem FOV 34 der PET-Vorrichtung an der den Patientenaufnahmebereich 11 umgebenden Umhausung 19 und die zweite Erfassungseinheit 42 direkt nach dem FOV 34 der PET-Vorrichtung an der den Patientenaufnahmebereich 11 umgebenden Umhausung 19 angeordnet. Weist die Positionsbestimmungseinheit 40 nur eine einzige Erfassungseinheit 41 auf, ist diese bevorzugt direkt neben dem FOV 34 der weiteren Bildgebungsvorrichtung 30, insbesondere der PET-Vorrichtung, an der den Patientenaufnahmebereich 11 umgebenden Umhausung 19 angeordnet. Insbesondere ist hierbei die Erfassungseinheit 41 direkt vor dem FOV 34 der PET-Vorrichtung an der den Patientenaufnahmebereich 11 umgebenden Umhausung 19 angeordnet.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Kombinierte Bildgebungsvorrichtung, die eine erste medizinische Bildgebungsvorrichtung, die als Magnetresonanzvorrichtung ausgebildet ist, und eine weitere medizinische Bildgebungsvorrichtung umfasst, mit einem Patientenaufnahmebereich, einer Patientenlagerungsvorrichtung, die einen in zumindest eine Richtung bewegbaren Patiententisch aufweist, und einer Positionsbestimmungseinheit zu einer Bestimmung einer Position des Patiententischs in zumindest eine Richtung,
**dadurch gekennzeichnet, dass** die Positionsbestimmungseinheit eine Erfassungseinheit, die an einer den Patientenaufnahmebereich umgebenden Umhausung angeordnet ist, und eine Positionskodiereinheit, die an dem Patiententisch angeordnet ist, aufweist, wobei die Erfassungseinheit zur Erfassung der Position des Patiententischs eine Positionsinformation der Positionskodiereinheit erfasst.

2. Magnetresonanzvorrichtung nach Anspruch 1,
**dadurch gekennzeichnet, dass** die Positionskodiereinheit ein Kodierband umfasst, das an einer Seite des Patiententischs angeordnet ist, wobei die Seite keine zur Lagerung eines Patienten ausgebildete Fläche umfasst.

3. Magnetresonanzvorrichtung nach einem der vorhergehenden Ansprüche,
**dadurch gekennzeichnet, dass** die Positionskodiereinheit eine Kodierung mit mehreren Kodierstrukturen umfasst, wobei die einzelnen Kodierstrukturen jeweils eine Positionsinformation in z-Richtung des Patiententischs und/oder eine Positionsinformation in eine weitere Richtung des Patiententischs umfassen.

4. Magnetresonanzvorrichtung nach Anspruch 3,
**dadurch gekennzeichnet, dass** die einzelnen Kodierstrukturen dabei jeweils eine Positionskodierung, die einem definierten Längenabschnitt am Patiententisch zugeordnet ist, und eine Informationskodierung umfassen.

5. Magnetresonanzvorrichtung nach Anspruch 4,
**dadurch gekennzeichnet, dass** ein Längenabschnitt des Patiententischs maximal 20 mm umfasst.

6. Magnetresonanzvorrichtung nach einem der Ansprüche 3 bis 5,
**dadurch gekennzeichnet, dass** die Kodierung einen Binärcode umfasst, wobei der Binärcode eine 8-Bit-Kodierung mit einer 2x4-Codematrix oder eine 9-Bit-Kodierung mit einer 3×3-Code-matrix oder eine 9-Bit-Kodierung mit einer 3x4-Codematrix umfasst.

7. Magnetresonanzvorrichtung nach einem der Ansprüche 3 bis 6,
**dadurch gekennzeichnet, dass** die Kodierung zusätzlich einen Richtungscode und/oder einen Markierungscode und/oder einen Paritätscode umfasst.

8. Magnetresonanzvorrichtung nach einem der vorhergehenden Ansprüche,
**dadurch gekennzeichnet, dass** die Erfassungseinheit eine Kamera umfasst, wobei die Kamera eine Auflösung von mindestens 400 Pixeln in eine Erfassungsrichtung umfasst.

9. Magnetresonanzvorrichtung nach Anspruch 8,
**dadurch gekennzeichnet, dass** die Erfassungseinheit weiterhin umfasst:
- eine Erfassungsoptik mit einem Prisma, wobei die Erfassungsoptik der Kamera vorgeschaltet angeordnet ist, und/oder
- zumindest eine LED und/oder
- zumindest einen Laser und/oder
- eine Steuereinheit.

10. Magnetresonanzvorrichtung nach einem der vorhergehenden Ansprüche,
**dadurch gekennzeichnet, dass** die Erfassungseinheit ein Schirmgehäuse mit einem Sichtfenster umfasst, wobei das Sichtfenster an einer dem Patientenaufnahmebereich und/oder dem Patiententisch zugewandten Seite des Schirmgehäuses angeordnet ist.

11. Magnetresonanzvorrichtung nach einem der vorhergehenden Ansprüche,
**dadurch gekennzeichnet, dass** die Erfassungseinheit zu einer Erfassung einer Position in zumindest zwei Richtungen des Patiententischs ausgebildet ist.

12. Magnetresonanzvorrichtung nach einem der vorhergehenden Ansprüche,
**dadurch gekennzeichnet, dass** die Positionsbestimmungseinheit zumindest eine Erfassungseinheit aufweist, wobei die zumindest eine Erfassungseinheit direkt an ein Field of View der weiteren Bildgebungsvorrichtung angrenzend an der den Patientenaufnahmebereichs umgebenden Umhausung angeordnet ist.

13. Magnetresonanzvorrichtung nach einem der vorhergehenden Ansprüche,
**dadurch gekennzeichnet, dass** die Positionsbestimmungseinheit zumindest zwei Erfassungseinheiten aufweist, wobei eine der Erfassungseinheiten in einem Eingangsbereich des Patientenaufnahmebereichs an der den Patientenaufnahmebereich umgebenden Umhausung angeordnet ist.

14. Magnetresonanzvorrichtung nach einem der vorhergehenden Ansprüche, **dadurch gekennzeichnet, dass** die zweite Bildgebungsvorrichtung eine PET-Vorrichtung und/oder eine Röntgenaufnahmevorrichtung ist.
